(19) Europäisches Patentamt
European Patent Office
Office européen des brevets

(11) EP 2 161 027 A2

(12) **EUROPEAN PATENT APPLICATION**

(43) Date of publication:
10.03.2010 Bulletin 2010/10

(51) Int Cl.:
*A61K 38/00* *(2006.01)*

(21) Application number: 09003133.7

(22) Date of filing: 22.12.2004

(84) Designated Contracting States:
AT BE BG CH CY CZ DE DK EE ES FI FR GB GR
HU IE IS IT LI LT LU MC NL PL PT RO SE SI SK TR

(30) Priority: 22.12.2003 US 530899 P

(62) Document number(s) of the earlier application(s) in
accordance with Art. 76 EPC:
04806688.0 / 1 696 943

(71) Applicant: YEDA RESEARCH AND
DEVELOPMENT CO., LTD.
76100 Rehovot (IL)

(72) Inventor: Shai, Yechiel
56333 Yehud (IL)

(74) Representative: Becker Kurig Straus
Patentanwälte
Bavariastrasse 7
80336 München (DE)

Remarks:
This application was filed on 04-03-2009 as a
divisional application to the application mentioned
under INID code 62.

(54) **Diastereomeric peptides useful as inhibitors of membrane protein assembly**

(57) The present invention relates to membrane binding diastereomeric peptides comprising amino acid sequences corresponding to a fragment of a transmembrane proteins, wherein at least two amino acid residues of the diastereomeric peptides being in a D-isomer configuration. The diastereomeric peptides are useful in inhibiting fusion membrane protein events, including specifically viral replication and transmission.

SEQUENCE LISTING

<110> Yeda Research and Development Co. Ltd. at the Weizmann
Institute of Science

<120> Diastereomeric Peptides Useful As Inhibitors of Membrane Protein
Assembly

<130> YEDA/038 PCT

<150> US 60/530,899
<151> 2003-12-22

<160> 29

<170> PatentIn version 3.3

<210> 1
<211> 36
<212> PRT
<213> Artificial sequence

<220>
<223> Synthetic peptide

<400> 1

Tyr Thr Ser Leu Ile His Ser Leu Ile Glu Glu Ser Gln Asn Gln Gln
1               5                   10                  15


Glu Lys Asn Glu Gln Glu Leu Leu Glu Leu Asp Lys Trp Ala Ser Leu
            20                  25                  30


Trp Asn Trp Phe
            35


<210> 2
<211> 36
<212> PRT
<213> Artificial sequence

<220>
<223> Synthetic peptide

<220>
<221> MISC_FEATURE
<222> (12)..(12)
<223> D-Ser

<220>
<221> MISC_FEATURE
<222> (13)..(13)
<223> D-Gln

<400> 2

Tyr Thr Ser Leu Ile His Ser Leu Ile Glu Glu Ser Gln Asn Gln Gln
1               5                   10                  15


Glu Lys Asn Glu Gln Glu Leu Leu Glu Leu Asp Lys Trp Ala Ser Leu
            20                  25                  30

Trp Asn Trp Phe
                35

<210>   3
<211>   36
<212>   PRT
<213>   Artificial sequence

<220>
<223>   Synthetic peptide


<220>
<221>   MISC_FEATURE
<222>   (32)..(32)
<223>   D-Leu

<220>
<221>   MISC_FEATURE
<222>   (33)..(33)
<223>   D-Trp

<400>   3

Tyr Thr Ser Leu Ile His Ser Leu Ile Glu Glu Ser Gln Asn Gln Gln
1               5                   10                  15


Glu Lys Asn Glu Gln Glu Leu Leu Glu Leu Asp Lys Trp Ala Ser Leu
            20                  25                  30


Trp Asn Trp Phe
                35


<210>   4
<211>   33
<212>   PRT
<213>   Artificial sequence

<220>
<223>   Synthetic peptide

<400>   4

Ala Val Gly Ile Gly Ala Leu Phe Leu Gly Phe Leu Gly Ala Ala Gly
1               5                   10                  15


Ser Thr Met Gly Ala Arg Ser Met Thr Leu Thr Val Gln Ala Arg Gln
            20                  25                  30


Leu


<210>   5
<211>   33
<212>   PRT
<213>   Artificial sequence

<220>
<223>   Synthetic peptide

```
<220>
<221>   MISC_FEATURE
<222>   (4)..(4)
<223>   D-Ile

<220>
<221>   MISC_FEATURE
<222>   (8)..(8)
<223>   D-Phe

<220>
<221>   MISC_FEATURE
<222>   (11)..(11)
<223>   D-Phe

<220>
<221>   MISC_FEATURE
<222>   (14)..(14)
<223>   D-Ala

<400>   5
```

Ala Val Gly Ile Gly Ala Leu Phe Leu Gly Phe Leu Gly Ala Ala Gly
1               5               10              15


Ser Thr Met Gly Ala Arg Ser Met Thr Leu Thr Val Gln Ala Arg Gln
            20              25              30


Leu


```
<210>   6
<211>   33
<212>   PRT
<213>   Artificial sequence

<220>
<223>   Synthetic peptide

<220>
<221>   MISC_FEATURE
<222>   (8)..(8)
<223>   D-Phe

<220>
<221>   MISC_FEATURE
<222>   (11)..(11)
<223>   D-Phe

<400>   6
```

Ala Val Gly Ile Gly Ala Leu Phe Leu Gly Phe Leu Gly Ala Ala Gly
1               5               10              15


Ser Thr Met Gly Ala Arg Ser Met Thr Leu Thr Val Gln Ala Arg Gln
            20              25              30


Leu


```
<210>   7
```

21

```
<211>   33
<212>   PRT
<213>   Artificial sequence

<220>
<223>   Synthetic peptide


<220>
<221>   MISC_FEATURE
<222>   (8)..(8)
<223>   D-Phe

<220>
<221>   MISC_FEATURE
<222>   (11)..(11)
<223>   D-Phe

<220>
<221>   MISC_FEATURE
<222>   (21)..(21)
<223>   D-Ala

<220>
<221>   MISC_FEATURE
<222>   (26)..(26)
<223>   D-Leu

<400>   7

Ala Val Gly Ile Gly Ala Leu Phe Leu Gly Phe Leu Gly Ala Ala Gly
1               5               10              15


Ser Thr Met Gly Ala Arg Ser Met Thr Leu Thr Val Gln Ala Arg Gln
            20              25              30


Leu



<210>   8
<211>   15
<212>   PRT
<213>   Artificial sequence

<220>
<223>   Synthetic peptide

<400>   8

Ile Thr Leu Ile Ile Phe Gly Val Met Ala Gly Val Ile Gly Thr
1               5               10              15


<210>   9
<211>   17
<212>   PRT
<213>   Artificial sequence

<220>
<223>   Synthetic peptide

<400>   9

Lys Lys Ile Thr Leu Ile Ile Phe Gly Val Met Ala Gly Val Ile Gly
1               5               10              15
```

Thr

```
<210>  10
<211>  19
<212>  PRT
<213>  Artificial sequence

<220>
<223>  Synthetic peptide

<400>  10
```

Lys Lys Ile Thr Leu Ile Ile Phe Gly Val Met Ala Gly Val Ile Gly
1                   5                   10                  15

Thr Lys Lys

```
<210>  11
<211>  15
<212>  PRT
<213>  Artificial sequence

<220>
<223>  Synthetic peptide


<220>
<221>  MISC_FEATURE
<222>  (8)..(8)
<223>  D-Val

<220>
<221>  MISC_FEATURE
<222>  (12)..(12)
<223>  D-Val

<400>  11
```

Ile Thr Leu Ile Ile Phe Gly Val Met Ala Gly Val Ile Gly Thr
1                   5                   10                  15

```
<210>  12
<211>  17
<212>  PRT
<213>  Artificial sequence

<220>
<223>  Synthetic peptide


<220>
<221>  MISC_FEATURE
<222>  (10)..(10)
<223>  D-Val

<220>
<221>  MISC_FEATURE
<222>  (14)..(14)
<223>  D-Val
```

<400> 12

Lys Lys Ile Thr Leu Ile Ile Phe Gly Val Met Ala Gly Val Ile Gly
1               5                   10                  15

Thr


<210> 13
<211> 19
<212> PRT
<213> Artificial sequence

<220>
<223> Synthetic peptide


<220>
<221> MISC_FEATURE
<222> (10)..(10)
<223> D-Val

<220>
<221> MISC_FEATURE
<222> (14)..(14)
<223> D-Val

<400> 13

Lys Lys Ile Thr Leu Ile Ile Phe Gly Val Met Ala Gly Val Ile Gly
1               5                   10                  15

Thr Lys Lys


<210> 14
<211> 15
<212> PRT
<213> Artificial sequence

<220>
<223> Synthetic peptide


<220>
<221> MISC_FEATURE
<222> (5)..(5)
<223> D-Ile

<220>
<221> MISC_FEATURE
<222> (13)..(13)
<223> D-Ile

<400> 14

Ile Thr Leu Ile Ile Phe Gly Val Met Ala Gly Val Ile Gly Thr
1               5                   10                  15


<210> 15
<211> 17
<212> PRT
<213> Artificial sequence

```
<220>
<223>  Synthetic peptide


<220>
<221>  MISC_FEATURE
<222>  (7)..(7)
<223>  D-Ile

<220>
<221>  MISC_FEATURE
<222>  (15)..(15)
<223>  D-Ile

<400>  15

Lys Lys Ile Thr Leu Ile Ile Phe Gly Val Met Ala Gly Val Ile Gly
1               5                   10                  15


Thr


<210>  16
<211>  19
<212>  PRT
<213>  Artificial sequence

<220>
<223>  Synthetic peptide


<220>
<221>  MISC_FEATURE
<222>  (7)..(7)
<223>  D-Ile

<220>
<221>  MISC_FEATURE
<222>  (15)..(15)
<223>  D-Ile

<400>  16

Lys Lys Ile Thr Leu Ile Ile Phe Gly Val Met Ala Gly Val Ile Gly
1               5                   10                  15


Thr Lys Lys


<210>  17
<211>  32
<212>  PRT
<213>  Artificial sequence

<220>
<223>  Synthetic peptide


<220>
<221>  MISC_FEATURE
<222>  (13)..(13)
<223>  D-Val
```

```
<220>
<221>  MISC_FEATURE
<222>  (17)..(17)
<223>  D-Val

<400>  17
```

Phe Ser Glu Pro Glu Ile Thr Leu Ile Ile Phe Gly Val Met Ala Gly
1               5                   10              15

Val Ile Gly Thr Ile Leu Leu Ile Ser Tyr Gly Ile Arg Arg Leu Ile
            20              25              30

```
<210>  18
<211>  35
<212>  PRT
<213>  Artificial sequence

<220>
<223>  Synthetic peptide


<220>
<221>  MISC_FEATURE
<222>  (16)..(16)
<223>  D-Val

<220>
<221>  MISC_FEATURE
<222>  (20)..(20)
<223>  D-Val

<400>  18
```

Lys Lys Lys Phe Ser Glu Pro Glu Ile Thr Leu Ile Ile Phe Gly Val
1               5                   10              15

Met Ala Gly Val Ile Gly Thr Ile Leu Leu Ile Ser Tyr Gly Ile Arg
            20              25              30

Arg Leu Ile
        35

```
<210>  19
<211>  32
<212>  PRT
<213>  Artificial sequence

<220>
<223>  Synthetic peptide


<220>
<221>  MISC_FEATURE
<222>  (10)..(10)
<223>  D-Ile

<220>
<221>  MISC_FEATURE
<222>  (18)..(18)
<223>  D-Ile

<400>  19
```

```
Phe Ser Glu Pro Glu Ile Thr Leu Ile Ile Phe Gly Val Met Ala Gly
1               5                   10              15

Val Ile Gly Thr Ile Leu Leu Ile Ser Tyr Gly Ile Arg Arg Leu Ile
            20              25              30
```

<210>    20
<211>    16
<212>    PRT
<213>    Artificial sequence

<220>
<223>    Synthetic peptide

<400>    20

```
Met Val Leu Gly Val Phe Ala Leu Leu Gln Leu Ile Ser Gly Ser Leu
1               5                   10              15
```

<210>    21
<211>    21
<212>    PRT
<213>    Artificial sequence

<220>
<223>    Synthetic peptide

<400>    21

```
Lys Lys Lys Met Val Leu Gly Val Phe Ala Leu Leu Gln Leu Ile Ser
1               5                   10              15

Gly Ser Leu Lys Lys
            20
```

<210>    22
<211>    16
<212>    PRT
<213>    Artificial sequence

<220>
<223>    Synthetic peptide

<220>
<221>    MISC_FEATURE
<222>    (10)..(10)
<223>    D-Gln

<220>
<221>    MISC_FEATURE
<222>    (15)..(15)
<223>    D-Ser

<400>    22

```
Met Val Leu Gly Val Phe Ala Leu Leu Gln Leu Ile Ser Gly Ser Leu
1               5                   10              15
```

<210>    23
<211>    21

```
<212>   PRT
<213>   Artificial sequence

<220>
<223>   Synthetic peptide


<220>
<221>   MISC_FEATURE
<222>   (13)..(13)
<223>   D-Gln

<220>
<221>   MISC_FEATURE
<222>   (18)..(18)
<223>   D-Ser

<400>   23
```

Lys Lys Lys Met Val Leu Gly Val Phe Ala Leu Leu Gln Leu Ile Ser
1               5                   10                  15

Gly Ser Leu Lys Lys
                20

```
<210>   24
<211>   8
<212>   PRT
<213>   Artificial sequence

<220>
<223>   Synthetic peptide

<400>   24
```

Ala Val Gly Ile Gly Ala Leu Phe
1               5

```
<210>   25
<211>   17
<212>   PRT
<213>   Artificial sequence

<220>
<223>   Synthetic peptide


<220>
<221>   MISC_FEATURE
<222>   (5)..(5)
<223>   D-Ala

<220>
<221>   MISC_FEATURE
<222>   (6)..(6)
<223>   D-Glu

<220>
<221>   MISC_FEATURE
<222>   (7)..(7)
<223>   D-Ala

<220>
<221>   MISC_FEATURE
<222>   (8)..(8)
```

```
<223>  D-Ala

<220>
<221>  MISC_FEATURE
<222>  (11)..(11)
<223>  D-Ala

<220>
<221>  MISC_FEATURE
<222>  (15)..(15)
<223>  D-Ala

<220>
<221>  MISC_FEATURE
<222>  (16)..(16)
<223>  D-Ala

<220>
<221>  MISC_FEATURE
<222>  (17)..(17)
<223>  D-Ala

<400>  25

Lys Lys Ile Thr Ala Gly Ala Ala Gly Val Ala Ala Gly Val Ala Ala
1               5                   10                  15

Ala


<210>  26
<211>  34
<212>  PRT
<213>  Artificial sequence

<220>
<223>  Synthetic peptide

<400>  26

Trp Met Glu Trp Asp Arg Glu Ile Asn Asn Tyr Thr Ser Leu Ile His
1               5                   10                  15

Ser Leu Ile Glu Glu Ser Gln Asn Gln Gln Glu Lys Asn Glu Gln Glu
                20                  25                  30

Leu Leu


<210>  27
<211>  13
<212>  PRT
<213>  Artificial sequence

<220>
<223>  Synthetic peptide

<400>  27

Ala Val Gly Ile Gly Ala Leu Phe Leu Gly Phe Leu Gly
1               5                   10
```

```
<210>  28
<211>  33
<212>  PRT
<213>  Artificial sequence

<220>
<223>  Synthetic peptide

<400>  28

Phe Phe Gly Ala Val Ile Gly Thr Ile Ala Leu Gly Val Ala Thr Ser
1               5                   10                  15


Ala Gln Ile Thr Ala Gly Ile Ala Leu Ala Glu Ala Arg Glu Ala Lys
            20                  25                  30


Arg



<210>  29
<211>  21
<212>  PRT
<213>  Artificial sequence

<220>
<223>  Synthetic peptide

<400>  29

Lys Lys Lys Met Val Leu Gly Val Phe Ala Leu Leu Phe Leu Ile Gly
1               5                   10                  15


Gly Ser Leu Lys Lys
            20
```

**Description**

**FIELD OF THE INVENTION**

The present invention relates to synthetic diastereomeric peptides derived from fragments of transmembrane proteins capable of inhibiting assembly of these transmembrane proteins. More particularly, the present invention relates to synthetic diastereomeric peptides, to pharmaceutical compositions comprising same and to methods of use thereof for treating diseases or disorders by preventing membrane protein assembly.

**BACKGROUND OF THE INVENTION**

Protein recognition within membranes is crucial for a wide variety of processes in all organisms. Protein recognition is demonstrated, for example, by inter-subunit association of receptors, ion channels, transporters and pumps. Many of these biologically important membrane proteins associate via their transmembrane domains. It was shown that reconstitution of a functional bacteriorhodopsin and lactose permease can be obtained from separate transmembrane segments. Other proteins that form hetero-oligomeric complexes are the T cell receptor and the MHC II complex. Viral fusion proteins such as influenza hemagglutinin and Hepatitis E1/E2, and the cellular fusion protein synaptobrevin also seem to oligomerize through their transmembrane domains. M13 major coat protein, phospholambdan, and Glycophorin A (GPA) are additional membrane proteins that were shown to oligomerize via their transmembrane domains.

The assembly and oligomerization of proteins within the membrane can be categorized into three main groups based on their orientation: (i) Transmembrane orientation; (ii) Surface orientation; and (iii) Oblique orientation. All these categories contain proteins having helical or beta sheet secondary structures or a combination of several structures.

The formation of active membrane complexes can be inhibited by specific interaction of the membrane proteins with synthetic peptides. This has been demonstrated by the following examples: (i) *Transmembrane orientation* - A short mutant helical peptide from delta-endotoxin was shown to abolish the pore-forming activity of the transmembrane pore hairpin. However, other mutants having only one amino acid difference were incapable of recognizing the hairpin (Gerber D., and Shai Y., 2000, J. Biol. Chem. 275: 23602-23607). The assembly of a Glycophorin A peptide having all L-amino acids with the wild type transmembrane Glycophorin A *in vivo* was also demonstrated (Gerber D., and Shai, Y., 2001, J. Biol. Chem. 276: 31229-31232). The recognition process of the Glycophorin A peptide within the membrane was sequence specific. Interestingly, the overall chirality of the peptide was not important for the recognition process as demonstrated by the fact that there was specific recognition within the membrane between Glycophorin A peptide having all D-amino acids and the wild type transmembrane domain, *in vivo* (Gerber D., and Shai Y., 2002, J. Mol. Biol. 322: 491-495); (ii) *Surface orientation* - two overlapping segments derived from HIV1 gp41 termed C34 and DP178 were previously shown to be competent inhibitors of viral fusion (Wild C., et al., 1993, AIDS Res. Hum. Retrov. 9: 1051-1053; Lu, M., and Kim, P. S., 1997, J. Biomol. Struct. Dynam. 15: 465-471). These two peptides were 100-1000 fold less active against HIV-2 strains. It was recently demonstrated that one of the major pathways through which DP178 inhibits fusion is through assembly with gp41 within the cellular membrane, arresting the fusion process in midway (Kliger, Y., et al., 2001, J. Biol. Chem. 276: 1391-1397); (iii) *Oblique orientation* - The HIV fusion peptide at the N-terminus of gp41 is known to inhibit viral fusion. The evidence collected points to a mechanism involving assembly within the membrane of the target cells (Kliger, Y., et al., 1997, J. Biol. Chem. 272: 13496-13505). Similarly to the transmembrane Glycophorin A, the fusion peptide of HIV-1 exhibits an achiral nature as the all-D fusion peptide showed similar inhibitory effect as of the all-L fusion peptide while preserving the species specificity, namely the selectivity towards HIV-2 was 100 fold lower (Pritsker, M., et al., 1998, Proc. Natl. Acad. Sci. USA 95: 7287-8292).

U.S. Patent No. 5,464,933 discloses DP178, fragments, analogs, and homologs thereof having anti-retroviral activity, specifically towards HIV. U.S. Patent No. 5,464,933 claims DP178, a peptide corresponding to amino acids 638-673 of the HIV-1$_{LAI}$ gp41 protein and analogs thereof, to which a macromolecular carrier may be conjugated. The peptides disclosed in U.S. Patent No. 5,464,933 may have at least one amino acid residue in a D-isomer configuration, though no specific embodiment or guidance is provided for any peptide comprising at least one D-amino acid. U.S. Patent No. 6,133,418 claims DP178, analogs thereof and pharmaceutical compositions comprising same.

U.S. Patent No. 6,093,794 discloses peptides corresponding to an amino acid sequence of Epstein-Barr virus protein. The peptides having structural and/or amino acid motif similarity to DP178 and to DP107, a peptide corresponding to amino acids 558-595 of the HIV$_{LAI}$ gp41, were identified through computer algorithms. The peptides disclosed in U.S. Patent 6,093,794 may have at least one amino acid in a D-isomer configuration though no specific enablement or guidance is provided for any peptide comprising at least one D-amino acid.

Using the same sequence searches and computer algorithms disclosed in U.S. Patent No. 6,093,794, U.S. Patent Nos. 6,228,983; 6,017,536; 6,013,263; and 6,020,459 disclose peptides derived from human respiratory syncytial virus, simian immunodeficiency virus, measles virus, and from HIV-1 and HIV-2, all of which are suggested to exhibit anti-viral activity. The peptide analogs disclosed in U.S. Patent No. 6,020,459 are suggested to have at least one amino acid in the D-isomer configuration. U.S. Patent No. 6,518,013 discloses methods for inhibiting transmission of an Epstein-Barr virus to a cell comprising contacting the cell with a peptide derived from an Epstein-Barr virus protein, said peptide identified by the same sequence searches described above.

U.S. Patent No. 5,840,843 provides a synthetic HIV-1 based polypeptide, which comprises an amino acid sequence corresponding to amino acid residues 600-862, or a portion thereof comprising the sequence corresponding to amino acid residues 637-666 of the envelope glycoprotein of HIV-1$_{IIIB}$. Dimers or trimers of the 637-666 amino acid sequence are shown to inhibit viral replication, HIV-1 mediated cytopathogenesis and cell fusion at higher efficiency than the monomeric peptide.

Use of the peptides disclosed in prior art is associated with several major disadvantages. As being hydrophobic and comprising all L-amino acid residues, these peptides are not water-soluble and are highly susceptible to proteolytic degradation. In addition, being short proteins they elicit a detrimental immune system.

Thus, there remains an unmet need for peptides having inhibitory activity of membrane protein assembly, which are water-soluble, resistant to proteolytic degradation and essentially not immunogenic.

## SUMMARY OF THE INVENTION

The present invention provides diastereomeric peptides capable of inhibiting binding of transmembrane proteins within the cell membrane and thereby inhibiting membrane protein assembly. The diastereomeric peptides exhibit low immunogenicity, are less susceptible to proteolytic degradation, and are more water-soluble than their native counterparts.

The present invention further provides diastereomeric peptides having at least one of the following biological activities: anti-fusogenic activity, anti-viral activity, anti-chemotactic activity and/or inhibitory activity of intracellular processes involving membrane protein assembly.

It is now disclosed for the first time that a diastereomeric peptide comprising an amino acid sequence corresponding to a fragment of a transmembrane protein, wherein at least two amino acid residues are in the D-isomer configuration, is capable of binding to the transmembrane protein and thereby inhibiting membrane protein interactions and membrane fusion processes.

The recognition between a transmembrane domain of a membrane protein and a peptide within the cell membrane was believed to be dependent upon the secondary structure of both the protein and the peptide. Unexpectedly, it is now disclosed that diastereomeric peptides comprising an amino acid sequence corresponding to a fragment of a transmembrane protein inhibit membrane fusion events despite the disruption of the secondary structure of these peptide-membrane protein complexes.

The diastereomeric peptides are highly advantageous over all L- or all D-amino acid peptides having the same amino acid sequence because of their higher water solubility, lower immunogenicity, and lower susceptibility to proteolytic degradation. Such characteristics endow the diastereomeric peptides with higher efficacy and higher bioavailability than those of the all L or all D-amino acid peptides comprising the same amino acid sequence.

The principles of the invention are exemplified herein below by diastereomeric peptides corresponding to the amino acid sequence of four different transmembrane proteins: i) DP178, a 36-mer peptide corresponding to amino acids 638 to 673 of HIV-1$_{LAV1}$ envelope protein gp41; ii) a 33-mer peptide corresponding to amino acids 512 to 544 of HIV-1$_{LAV1}$ gp41; iii) a 15-mer Glycophorin A peptide corresponding to amino acids 92 to 106 of Glycophorin A; and iv) a peptide corresponding to amino acids 13 to 28 of the aspartate Tar receptor. The diastereomeric peptides were found to inhibit membrane fusion events at a similar or higher efficacy than their respective native counterparts comprising all L-amino acids.

The diastereomeric peptides exemplified in the present invention represent the three different categories of membrane protein assembly and oligomerization, specifically transmembrane orientation, surface orientation, and oblique orientation. It will be, therefore, understood that the present invention encompasses a wide variety of diastereomeric peptides comprising an amino acid sequence corresponding to a fragment of a transmembrane protein, wherein at least two amino acid residues within the peptide sequence are in D-isomer configuration. The diastereomeric peptides of the present invention are shown to bind to the transmembrane protein and thereby inhibit efficiently the assembly of said transmembrane protein.

According to one aspect, the present invention provides a membrane binding diastereomeric peptide comprising from about 7 to 50 amino acid residues corresponding to an amino acid sequence of a fragment of a transmembrane protein, wherein at least two amino acid residues of the diastereomeric peptide are of the D-isomer configuration, said diastereomeric peptide capable of binding the transmembrane protein thereby inhibiting functional assembly of said transmembrane protein, and active fragments, derivatives, analogs or salts thereof.

It will be understood that the diastereomeric peptides of the invention are not limited in size. However, the invention particularly contemplates peptides having fewer than about 50 amino acid residues in total. Additionally, the peptides of the invention should include at least 7 amino acid residues, which enable the peptide to be incorporated into a lipid bilayer. According to some preferred embodiments, the diastereomeric peptides of the invention comprise from 10 to 40 amino acid residues.

The present invention also contemplates polypeptides or proteins in which the core motif sequence, namely the amino acid sequence of the diastereomeric peptides of the present invention, is artificially implanted within a sequence of the polypeptide or protein.

According to the principles of the present invention, inhibitory activity of functional assembly of a transmembrane protein includes at least one biological activity selected from anti-fusogenic activity, anti-viral activity, anti-chemotactic activity and inhibitory activity of intracellular processes involving membrane protein assembly. In addition, according to the principles of the present invention, the number and location of the D-amino acid residues in a diastereomeric peptide of the invention may vary so long as the inhibition of the functional assembly of a transmembrane protein is maintained or preferably enhanced. It should be appreciated that the diastereomeric peptides of the present invention may further comprise one or more positively charged amino acid residues at either side of the peptide sequence, i.e., at the amino terminus, at the carboxy terminus, or at both termini.

According to certain embodiments, the diastereomeric peptide comprising the amino acid sequence of a fragment of a transmembrane protein is selected from the group consisting of viral proteins, bacterial proteins, ion channels, receptors, transporters, and pumps.

According to certain embodiments, the diastereomeric peptide comprises an amino acid sequence of a viral envelope surface glycoprotein. According to some embodiments, the viral envelope glycoprotein is HFV-1$_{LAV1}$ gp41. According to some exemplary embodiments, the diastereomeric peptide comprises the amino acid sequence of DP178 set forth in SEQ ID NO:1:

YTSLIHSLIEESQNQQEKNEQELLELDKWASLWNWF    (SEQ ID NO:1)

The present invention explicitly encompasses an active fragment, derivative, analog, or salt of the diastereomeric DP178 wherein the fragment, derivative, analog or salt thereof inhibits the functional assembly of the mV-1$_{LAV1}$ gp41.

According to certain embodiments, the diastereomeric peptide comprising SEQ ID NO: is selected from the group consisting of SEQ ID NO:2 and SEQ ID NO:3 (the D-amino acid residues are bold and underlined):

YTSLIHSLIEE**SQ**NQQEKNEQELLELDKWASLWNWF    (SEQ ID NO:2)
YTSLIHSLIEESQNQQEKNEQELLELDKWAS**LW**NWF    (SEQ ID NO:3)

According to additional embodiments, the diastereomeric peptide comprises an amino acid sequence corresponding to HIV-1$_{LAV1}$ gp41 amino terminal fusion peptide, a 33-mer peptide set forth in SEQ ID NO:4:

AVGIGALFLGFLGAAGSTMGARSMTLTVQARQL    (SEQ ID NO: 4)

The present invention explicitly encompasses an active fragment, derivative, analog, or salt of the diastereomeric HIV-1$_{LAV1}$ gp41 amino terminal fusion peptide wherein the fragment, derivative, analog or salt thereof inhibits the functional assembly of the HIV-1$_{LAV1}$ gp41.

According to certain embodiments, the diastereomeric peptide comprising SEQ ID NO:4 is selected from the group consisting of SEQ ID NO:5 to SEQ ID NO:7 (the D-amino acid residues are bold and underlined):

AVG**I**GAL**F**LG**F**LG**A**AGSTMGARSMTLTVQARQL    (SEQ ID NO: 5)
AVGIGAL**F**LG**F**LGAAGSTMGARSMTLTVQARQL    (SEQ ID NO: 6)
AVGIGAL**F**LG**F**LGAAGSTMG**A**RSMT**L**TVQARQL    (SEQ ID NO: 7)

According to additional embodiments, the diastereomeric peptide comprises an amino acid sequence corresponding to a transmembrane domain of Glycophorin A set forth in SEQ ID NO: 8:

ITLIIFGVMAGVIGT    (SEQ ID NO: 8)

The present invention explicitly encompasses an active fragment, derivative, analog or salt of the diastereomeric Glycophorin A peptide set forth in SEQ ID NO:8 wherein the fragment, derivative, analog or salt thereof inhibits the functional assembly of Glycophorin A.

According to some embodiments, the diastereomeric peptide comprising the amino acid sequence corresponding to the transmembrane domain of Glycophorin A set forth in SEQ ID NO:8 further comprises at least one positively charged amino acid residue at the amino terminus of the peptide, at the carboxy terminus, or at both. According to some exemplary embodiments, the diastereomeric peptide comprising SEQ ID NO: 8 is selected from the group consisting of SEQ ID NO: 9 and SEQ ID NO: 10:

<div style="text-align:center">

KKITLIIFGVMAGVIGT      (SEQ ID NO:9)

KKITLIIFGVMAGVIGTKK      (SEQ ID NO:10)

</div>

According to certain exemplary embodiments, the diastereomeric peptide comprising SEQ ID NO:8 is selected from the group consisting of SEQ ID NO:11 to SEQ ID NO:19 (the D-amino acid residues are bold and underlined):

<div style="text-align:center">

ITLIIFG**V**MAG**V**IGT      (SEQ ID NO: 11)

KKITLIIFG**V**MAG**V**IGT      (SEQ ID NO: 12)

KKITIIIFG**V**MAG**V**IGTKK      (SEQ ID NO: 13)

ITL**I**IFGVMAGV**I**GT      (SEQ ID NO: 14)

KKITL**I**IFGVMAGV**I**GT      (SEQ ID NO: 15)

KKITL**I**IFGVMAGV**I**GTKK      (SEQ ID NO: 16)

FSEPEITLIIFG**V**MAG**V**IGTILLISYGIRRLI      (SEQ ID NO: 17)

KKKFSEPEITLIIFG**V**MAGVIGTILLISYGIRRLI      (SEQ ID NO: 18)

FSEPEITL**I**IFGVMAGV**I**GTILLISYGIRRLI      (SEQ ID NO: 19)

</div>

According to some other embodiments, the present invention provides a diastereomeric peptide comprising the amino acid sequence corresponding to the transmembrane domain-1 of the aspartate Tar receptor set forth in SEQ ID NO:20:

<div style="text-align:center">

MVLGVFALLQLISGSL      (SEQ ID NO: 20)

</div>

The present invention explicitly encompasses an active fragment, a derivative, analog or a salt of the diastereomeric peptide corresponding to the transmembrane domain-1 of aspartate Tar receptor set forth in SEQ ID NO:20 wherein the fragment, derivative, analog or salt thereof inhibits the functional assembly of the aspartate Tar receptor.

According to some embodiments, the diastereomeric peptide comprising the amino acid sequence of the transmembrane domain of the aspartate Tar receptor set forth in SEQ ID NO:20 further comprises at least one positively charged amino acid residue at either or both sides of the peptide. According to some embodiments, the diastereomeric peptide comprises the amino acid sequence set forth in SEQ ID NO: 21:

<div style="text-align:center">

KKKMVLGVFALLQLISGSLKK      (SEQ ID NO:21)

</div>

According to certain exemplary embodiments, the diastereomeric peptide comprising SEQ ID NO:20 is selected from the group consisting of SEQ ID NO:22 and SEQ ID NO:23 (the D-amino acid residues are bold and underlined):

<div style="text-align:center">

MVLGVFALL**Q**LI**S**GSL      (SEQ ID NO:22)

KKKMVLGVFALL**Q**LISG**S**LKK      (SEQ ID NO:23)

</div>

According to another aspect, the present invention provides a pharmaceutical composition comprising as an active ingredient a membrane binding diastereomeric peptide comprising from about 7 to 50 amino acid residues corresponding to an amino acid sequence of a fragment of a transmembrane protein, wherein at least two amino acid residues of the diastereomeric peptide being in the D-isomer configuration, said diastereomeric peptide capable of binding the transmembrane protein thereby inhibiting functional assembly of said transmembrane protein, and active fragments, derivatives, or salts thereof, and a pharmaceutically acceptable carrier.

According to a further aspect, the present invention provides a method for inhibiting membrane protein assembly in a cell comprising contacting the cell with an effective amount of a membrane binding diastereomeric peptide according to the principles of the present invention, thereby inhibiting functional assembly of the membrane protein.

According to another aspect the present invention provides a method for inhibiting infection of a cell by a virus comprising contacting the cell with an effective amount of a membrane binding diastereomeric peptide according to the

<div style="text-align:center">5</div>

principles of the present invention, thereby inhibiting infection of the cell. According to some embodiments, the diastereomeric peptide be utilized for inhibiting infection by a human virus including, but not limited to, HIV, human T-lymphocyte virus, leukemia virus, human respiratory syncytial virus, human parainfluenza virus, influenza virus, measles virus, and Epstein-Barr virus. However, the present invention also encompasses inhibition of viral infection by non-human viruses including, but not limited to, bovine leucosis virus, feline sarcoma virus, feline leukemia virus, simian sarcoma virus, simian leukemia virus, simian immunodeficiency virus, canine distemper virus, Newcastle disease virus, simian Mason-Pfizer virus, and sheep progressive pneumonia virus.

According to another aspect the present invention provides a method for inhibiting chemotaxis of a cell comprising contacting the cell with an effective amount of a membrane binding diastereomeric peptide according to the principles of the present invention, thereby inhibiting chemotaxis of said cell. According to some embodiments, the method for inhibiting chemotaxis may be utilized for inhibiting nutrient chemotaxis of bacteria.

According to still a further aspect, the present invention provides a method for inhibiting virus replication or transmission in a subject comprising administering to the subject in need thereof a therapeutically effective amount of a pharmaceutical composition comprising as an active ingredient a membrane binding diastereomeric peptide according to the principles of the present invention, thereby inhibiting virus replication or transmission. According to some embodiments, the subject is human. According to additional embodiments, the diastereomeric peptide is utilized for inhibiting replication of a human virus including, but not limited to, HIV, human T-lymphocyte virus, human respiratory syncytial virus, human parainfluenza virus, influenza virus, measles virus, and Epstein-Barr virus. According to other embodiments, the subject is an animal. According to other embodiments, the diastereomeric peptide is utilized for inhibiting replication of an animal virus including, but not limited to, bovine leucosis virus, feline sarcoma virus, feline leukemia virus, simian sarcoma virus, simian leukemia virus, simian immunodeficiency virus, canine distemper virus, Newcastle disease virus, simian Mason-Pfizer virus, and sheep progressive pneumonia virus.

These and other embodiments of the present invention will be better understood in relation to the figures, description, examples, and claims that follow.

## BRIEF DESCRIPTION OF THE FIGURES

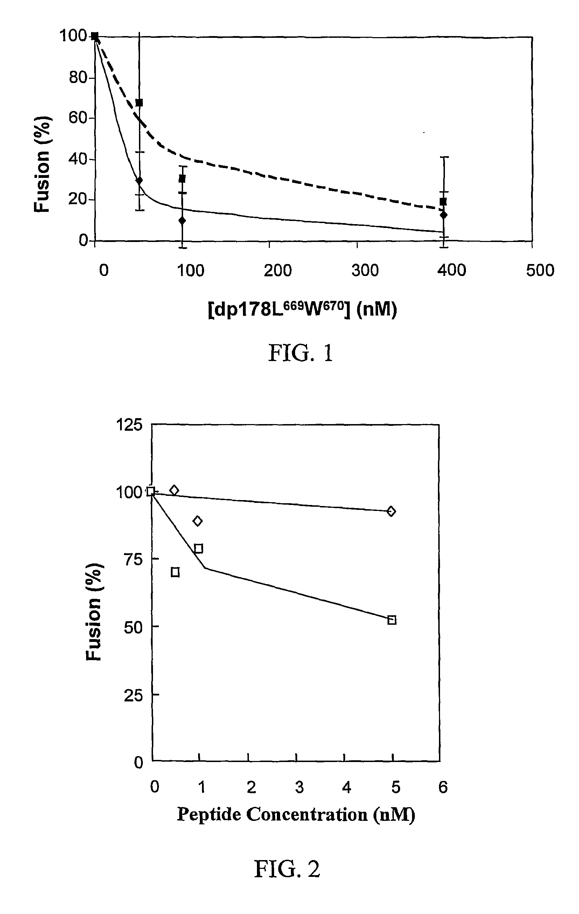

FIG. **1** shows the effect of [D-LW]-DP178 on the fusion of HIV-1 gp120-41 expressing TF228 cells with CD4+ CXCR4+ 3T3 mouse fibroblasts. Solute (♦) and lipid (■) refer to mixing of cell content and of cell membranes, respectively, by the DP178 diastereomeric peptide.

FIG. **2** shows the effect of a diastereomeric analog of the amino terminal fusion peptide derived from HIV-1$_{LAV1a}$ gp41 on the fusion of HIV-1 gp120-41 expressing TF228 cells with CD4+ CXCR4+ 3T3 mouse fibroblasts. The inhibitory effect of the diastereomeric peptide of SEQ ID NO:5 (□) is shown. An 8-mer wild-type peptide having the sequence AVGIGAL**F** set forth in SEQ ID NO:24 was derived from the N-terminal region of the fusion peptide (0) is shown as a control.

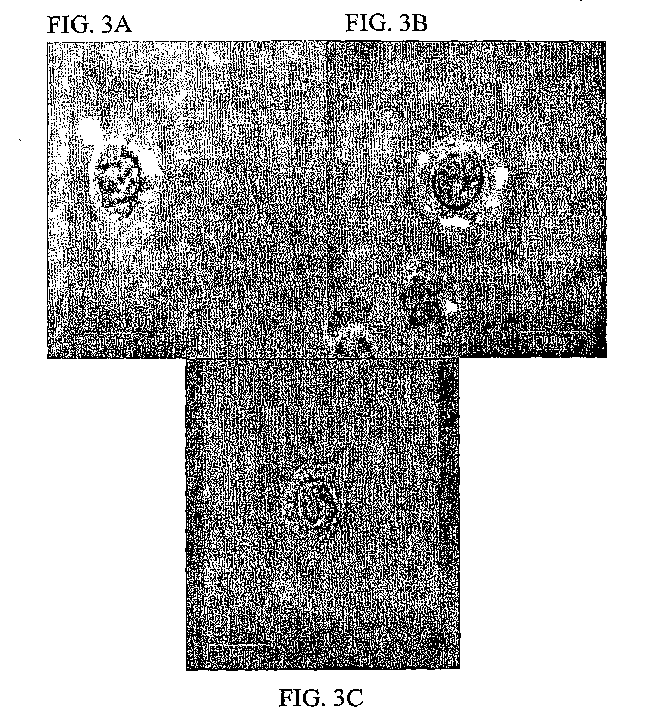

FIG. **3A-C** show the partition of the fusion peptide and its analog in T-cell membranes. Images show an overlay of fluorescence and transmission light. They are focused on the longitudinal section of T-cells. FIG. 3A, WT fusion peptide; FIG. 3B, IFFA fusion peptide of SEQ ID NO:5; FIG. 3C, Control cells without any peptide.

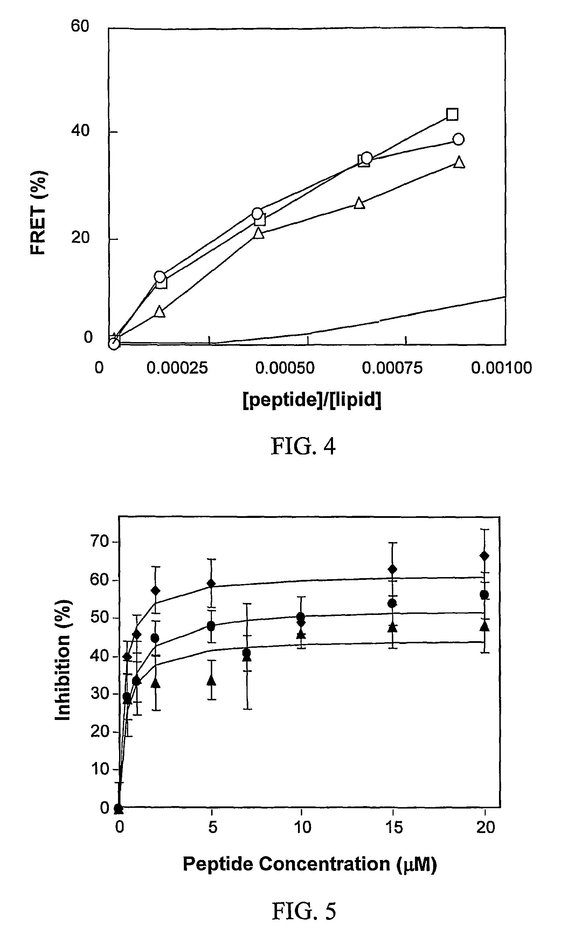

FIG. **4** shows fluorescence energy transfer (FRET) as a function of concentration of Rho-labeled fusion peptides. Transfer efficiencies between donor-WT and acceptor-WT (□), between donor-WT and acceptor-IFFA (o), and between donor-WT and acceptor-FF of SEQ ID NO:6 (Δ), are plotted versus the bound acceptor/lipid molar ratio. A theoretical plot showing energy transfer efficiency as a function of the surface density of the acceptors, assuming random distribution of donors and acceptors, and Ro=51Å, is given for comparison (line).

FIG. **5** shows the heterodimer formation through ToxR Glycophorin A (GPA) peptide inhibition. The results were normalized between wt-GPA homodimer as 0% inhibition and a 16-mer peptide of Ala (A$_{16}$ monomer) as 100% inhibition. All-L GPA peptide set forth in SEQ ID NO:9 (▲); All-D GPA peptide having the peptide sequence of SEQ ID NO:8 but all the residues are in the D-isomer configuration (e); and 2D-GPA peptide set forth in SEQ ID NO:12 (▲).

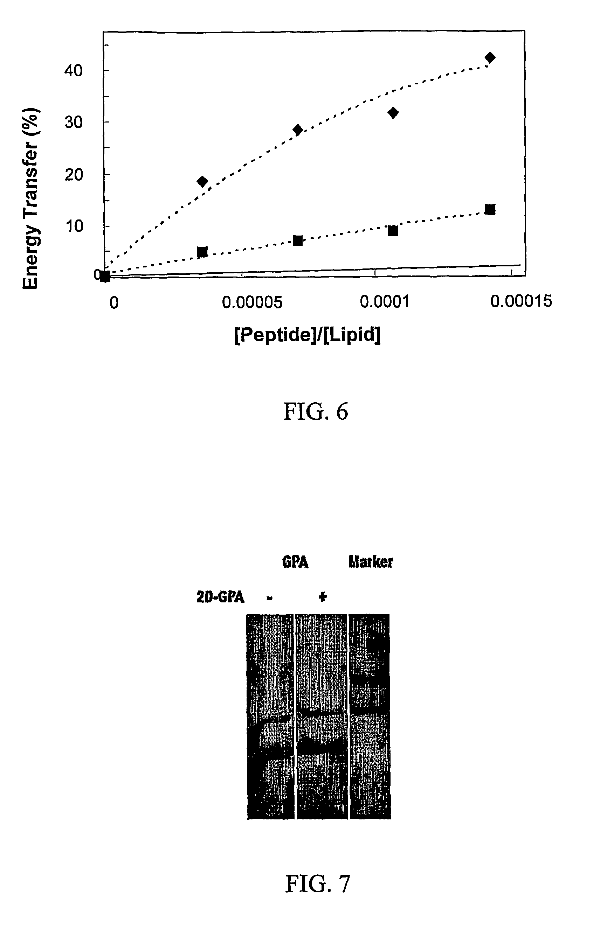

FIG. **6** shows the specific recognition between wt-Glycophorin A (GPA) and its diastereomer *in vitro.* Theoretically and experimentally derived percentages of energy transfer efficiencies between donor nitrobenzofurazane (NBD) labeled wt-GPA and acceptor Rhodamine labeled 2D-GPA of SEQ ID NO:11 are shown (♦); a negative control of donor NBD labeled membrane binding peptide that forms monomer in the membrane and acceptor A$_2$-GPA-Rhodamine having the sequence KKIT**AGAA**GV**A**AGV**AAA** set forth in SEQ ID NO:25 is also shown (■). The broken line represents a random distribution of monomers using a Ro of 51Å.

FIG. **7** shows the expression levels of ToxR Glycophorin A (GPA) in the presence or absence of 2D-GPA set forth in SEQ ID NO:11. Western blot analysis using anti maltose binding protein antibodies confirmed that the diastereomeric exogenous peptide did not interfere with the expression of the ToxR GPA construct.

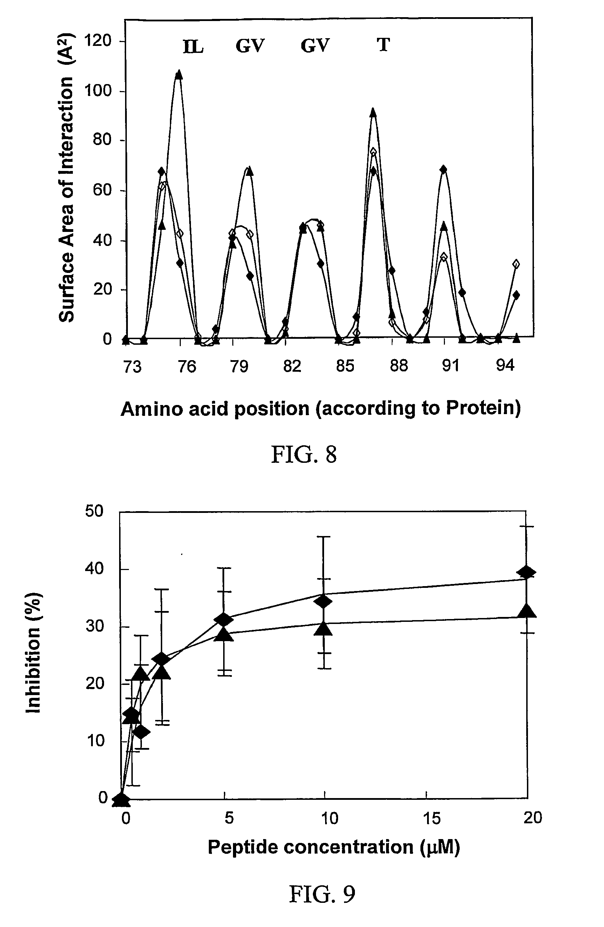

FIG. **8** presents the analysis of the structures resulting from molecular dynamics simulation. The contact surface area of single residues with the opposite helix was calculated with the CSU tool (Sobolev, V., et al., 1999, Bioinform. 15:327-332). The NMR model (▲); homodimer (♦); and heterodimer (0). Amino acids belonging to the *"interaction motif"* are marked as single letters.

FIG. **9** shows the inhibition of Tar-1 dimerization in the presence of Tar-1 all-L and its diastereomeric peptide. Dose response of β-galactosidase inhibition as a function of Tar-1 all-L (♦) or Tar-1 diastereomer (■).

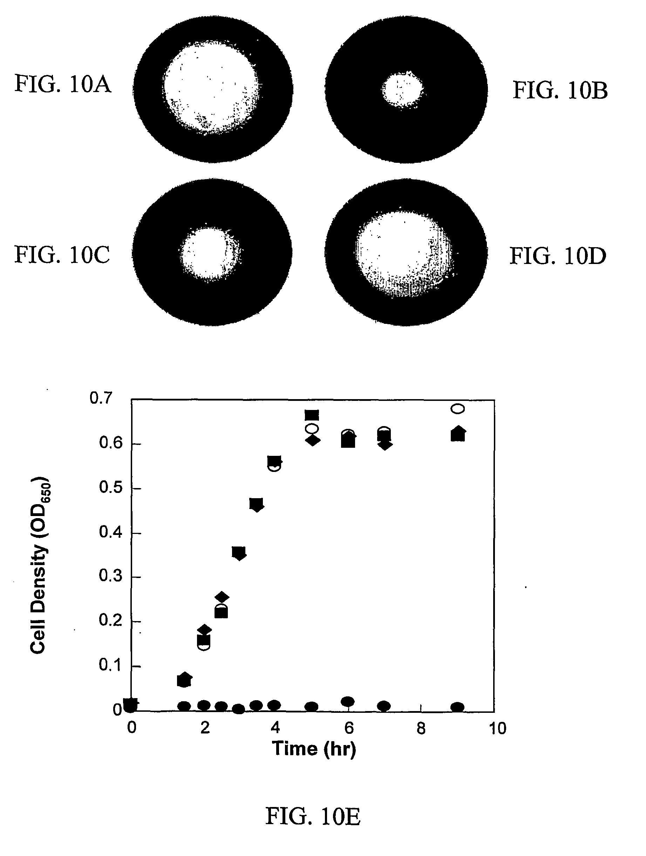

FIG. **10A-E** shows swarm assay on TB semisolid agar. FIG. 10A, Chemotactic response of the bacteria strain (CP362) that overexpresses the Tar protein as the only chemotaxis receptor (pNT201); FIG. 10B, Chemotactic response of the bacteria strain (CP362) without the Tar overexpressed plasmid; FIG. 10C, Chemotactic response of the bacteria stain of FIG. 10A in the presence of 20μM Tar-1 diastereomeric peptide; FIG. 10D, Chemotactic response of the bacteria stain of FIG. 10A in the presence of 20μM Tar-1 F/G mutant peptide; and FIG. 10E, Growth of CP362 containing pNT201 plasmid in the presence of either 20μM Tar-1 all-L peptide (♦), 20μM Tar-1 all-D peptide (■), in the absence of any peptide (o) and in the presence of chloramphenicol (●).

## DETAILED DESCRIPTION OF THE INVENTION

The present invention provides membrane binding diastereomeric peptides corresponding to a fragment of a transmembrane protein, the diastereomeric peptides comprise at least two amino acid residues in a D-isomer configuration being capable of binding to the transmembrane protein thereby inhibiting the functional assembly of said transmembrane protein. The diastereomeric peptides of the present invention are thus useful for inhibiting various biological events associated with membrane protein assembly.

The diastereomeric peptides are highly advantageous over all L- or all D-amino acid peptides having the same amino acid sequence because of their higher water solubility, lower immunogenicity (see, for example, Benkirane, N., et al., 1993, J. Biol. Chem. 268: 26279-26285), and lower susceptibility to proteolytic degradation. Such characteristics endow the diastereomeric peptides with higher efficacy and higher bioavailability than those of the all L or all D-amino acid peptides comprising the same amino acid sequence.

The term "diastereomeric peptide" as used herein refers to a peptide comprising both L-amino acid residues and D-amino acid residues. The number and position of D-amino acid residues in a diastereomeric peptide may be variable so long as the inhibitory activity of the peptide on functional assembly of a transmembrane protein is maintained or enhanced.

The diastereomeric peptides of the invention are not limited in size. However, the invention particularly contemplates peptides having fewer than about 50 amino acid residues in total. Additionally, the peptides of the invention should include at least 7 amino acid residues, which enable the peptide to be incorporated into a lipid bilayer. The present invention also contemplates polypeptides or proteins in which the core motif sequence, namely the amino acid sequence of the diastereomeric peptides of the present invention, is artificially implanted within a sequence of the polypeptide or protein.

The diastereomeric peptides of the invention may be synthesized or prepared by techniques well known in the art. The peptides can be synthesized by a solid phase peptide synthesis method of Merrifield (see J. Am. Chem. Soc., 85:2149, 1964). Alternatively, a diastereomeric peptide of the present invention can be synthesized using standard solution methods well known in the art (see, for example, Bodanszky, M., Principles of Peptide Synthesis, Springer-Verlag, 1984) or by any other method known in the art for peptide synthesis.

In general, these methods comprise sequential addition of one or more amino acids or suitably protected amino acids to a growing peptide chain bound to a suitable resin.

Normally, either the amino or carboxyl group of the first amino acid is protected by a suitable protecting group. The protected or derivatized amino acid can then be either attached to an inert solid support (resin) or utilized in solution by adding the next amino acid in the sequence having the complimentary (amino or carboxyl) group suitably protected, under conditions conductive for forming the amide linkage. The protecting group is then removed from this newly added amino acid residue and the next amino acid (suitably protected) is added, and so forth. After all the desired amino acids have been linked in the proper sequence, any remaining protecting groups are removed sequentially or concurrently, and the peptide chain, if synthesized by the solid phase method, is cleaved from the solid support to afford the final peptide.

In the solid phase peptide synthesis method, the alpha-amino group of the amino acid is protected by an acid or base sensitive group. Such protecting groups should have the properties of being stable to the conditions of peptide linkage formation, while being readily removable without destruction of the growing peptide chain. Suitable protecting groups are t-butyloxycarbonyl (BOC), benzyloxycarbonyl (Cbz), biphenylisopropyloxycarbonyl, t-amyloxycarbonyl, isobornyloxycarbonyl, (alpha,alpha)-dimethyl-3,5dimethoxybenzyloxycarbonyl, o-nitrophenylsulfenyl, 2-cyano-t-butyloxycarbonyl, 9-fluorenylmethyloxycarbonyl (FMOC) and the like. The BOC protecting group is preferred.

In the solid phase peptide synthesis method, the C-terminal amino acid is attached to a suitable solid support. Suitable solid supports useful for the above synthesis are those materials, which are inert to the reagents and reaction conditions of the stepwise condensation-deprotection reactions, as well as being insoluble in the solvent media used. Suitable solid supports are chloromethylpolystyrene-divinylbenzene polymer, hydroxymethyl-polystyrene-divinylbenzene polymer, and the like. The coupling reaction is accomplished in a solvent such as ethanol, acetonitrile, N,N-dimethylformamide (DMF), and the like. The coupling of successive protected amino acids can be carried out in an automatic polypeptide synthesizer as is well known in the art. One specific procedure is described herein below in Example 1.

The peptides of the invention may alternatively be synthesized such that one or more of the bonds, which link the amino acid residues of the peptides are non-peptide bonds. These alternative non-peptide bonds include, but are not limited to, imino, ester, hydrazide, semicarbazide, and azo bonds, which can be formed by reactions well known to skilled in the art.

It should be understood that a diastereomeric peptide of the invention need not be identical to the amino acid sequence of a naturally occurring membrane protein so long as it includes the required sequence that allows it to be incorporated into a lipid bilayer and as such is able to inhibit membrane protein assembly.

The term "membrane binding" diastereomeric peptide is used throughout the present specification and claims to refer to a peptide capable of interacting or binding to a membrane protein.

The term "functional assembly" of a transmembrane protein is used throughout the present specification and claims to refer to complex formation or non-covalent interaction between transmembrane proteins, which lead to membrane fusion events and/or to intracellular processes initiated by the membrane protein complex formation or membrane protein interactions. The term "membrane protein" is used herein to refer to cellular membrane proteins of human or non-human cells as well as to viral envelope proteins. It should be understood that functional assembly of a protein includes homodimerization and heterodimerization, i.e., the protein may interact with an identical protein or it may interact with a different protein. Thus, functional assembly includes, but is not limited to, an interaction between two proteins adjacent to each other to form a non-covalent complex within the same cellular membrane and an interaction between different membrane proteins present in different cells. The terms "functional assembly of a membrane protein" and "membrane protein assembly" are used interchangeably throughout the specification and claims. The term "transmembrane protein" refers to a membrane protein that spans the lipid bilayer of the membrane.

The present invention encompasses diastereomeric peptide derivatives and analogs having amino acid deletions, substitutions, and/or extensions.

The amino acid substitutions may be of conserved or non-conserved nature. Conserved amino acid substitutions consist of replacing one or more amino acids of a diastereomeric peptide of the invention with amino acids of similar charge, size, and/or hydrophobicity characteristics, such as, for example, substitution of a glutamic acid (E) to aspartic acid (D). Non-conserved substitutions consist of replacing one or more amino acids of a diastereomeric peptide with amino acids possessing dissimilar charge, size, and/or hydrophobicity characteristics, such as, for example, substitution of a glutamic acid (E) to valine (V). The amino acid substitutions may also include non-natural amino acids.

Amino acid extensions may consist of a single amino acid residue or stretches of residues. The extensions may be made at the carboxy or amino terminal end of a diastereomeric peptide, as well as at a position internal to the peptide. Such extensions will generally range from 2 to 15 amino acids in length. Preferably, the peptide comprises not more than 50 amino acid residues in total. It is contemplated that extensions made at either the carboxy or amino terminus of the peptide of interest may be of a broader size range, with about 2 to about 20 amino acids being preferred. One or more such extensions may be introduced into a peptide so long as such extensions result in a peptide, which still exhibits inhibitory activity of membrane protein assembly. According to some preferred embodiments, the extension of the diastereomeric peptides of the invention comprise at least one positively charged amino acid at the amino terminus, at the carboxy terminus, or at both termini of the peptide. It should be understood that addition of one or more positively charged amino acid residues to the diastereomeric peptide improves its solubility in aqueous solutions while retaining its inhibitory activity of membrane protein assembly. Positively charged amino acids that may be are added to the diastereomeric peptides of the invention include, but are not limited to, lysine, arginine, histidine, or any other non-charged amino acid derivatized to yield a positively charged amino acid by, for example, methylation or by any other derivatization method known in the art.

The present invention encompasses analogs of the diastereomeric peptides, said analogs may comprise at the amino terminus of the peptide a group such as, for example, acetyl group, a 9-fluorenylmethoxy-carbonyl group, or a hydrophobic group, and/or at the carboxy terminus of the peptide a group such as, for example, an amido group, or a hydrophobic group, so that the stability, bioavailability, and/or inhibitory activity of the peptides are retained or preferably enhanced.

Deletions in a diastereomeric peptide are also within the scope of the invention. Such deletions consist of the removal of one or more amino acid residues from a naturally occurring fragment of a transmembrane protein, with the lower limit length of the resulting peptide sequence being 7 amino acids. Such deletions may involve a single contiguous or greater than one discrete portion of the transmembrane protein. One or more such deletions may be introduced so long as such deletions result in peptides, which still exhibit inhibitory activity of membrane protein assembly.

The present invention also encompasses conjugates comprising the diastereomeric peptides of the invention and a carrier protein so long as the conjugate is capable of inhibiting membrane protein assembly.

Typically, the present invention encompasses derivatives of the diastereomeric peptides. The term "derivative" includes any chemical derivative of the peptide having one or more residues chemically derivatized by reaction of side chains or functional groups. Such derivatized molecules include, for example, those molecules in which free amino groups have been derivatized to form amine hydrochlorides, p-toluene sulfonyl groups, carbobenzoxy groups, t-butyloxycarbonyl groups, chloroacetyl groups or formyl groups. Free carboxyl groups may be derivatized to form salts, methyl and ethyl esters or other types of esters or hydrazides. Free hydroxyl groups may be derivatized to form O-acyl or O-alkyl derivatives. The imidazole nitrogen of histidine may be derivatized to form N-im-benzylhistidine. Also included as chemical derivatives are those peptides, which contain one or more naturally occurring amino acid derivatives of the twenty standard amino acid residues. For example: 4-hydroxyproline may be substituted for proline; 5-hydroxylysine may be substituted for lysine; 3-methylhistidine may be substituted for histidine; homoserine may be substituted or serine; and ornithine may be substituted for lysine.

The present invention provides diastereomeric peptides comprising from about 7 to 50 amino acid residues corresponding to a fragment of a transmembrane protein. Preferably, the diastereomeric peptides of the invention comprise from 10 to 40 amino acid residues. According to some embodiments, the diastereomeric peptides comprise the amino acid sequence of a transmembrane domain of a membrane protein. Typically, a transmembrane domain contains hydrophobic amino acid residues, which enable the transmembrane domain to be incorporated within a lipid bilayer. Examples of hydrophobic amino acids are alanine, leucine, isoleucine, proline, valine, and phenylalanine.

According to the invention, the membrane binding diastereomeric peptides exhibit inhibitory activity of functional assembly of a membrane protein. The inhibitory activity of functional assembly of a membrane protein includes, but is not limited to, anti-fusogenic activity, anti-viral activity, anti-chemotactic activity and ability to inhibit intracellular processes involving membrane protein assembly such as, for example, inhibitory activity of channel formation, and inhibitory activity of hormone signaling.

The term "anti-fusogenic" activity as used herein refers to inhibitory effect of membrane fusion events including, but not limited to, fusion of cells such as viral infected cells with non-infected cells and fusion of a viral structure with a cell membrane. Thus, a peptide exhibits an anti-fusogenic activity if the level of membrane fusion events between two or more moieties is lower in the presence of the peptide than in its absence. The moieties may be, for example, cell membranes or a viral structure such as a viral envelope.

Assays for cell fusion events are well known to those of skill in the art. Cell fusion assays are generally performed in vitro. Such an assay includes culturing cells, which, in the absence of any treatment, would undergo an observable level of syncytial formation. For example, uninfected cells may be incubated in the presence of cells chronically infected with a virus that induces cell fusion. Viruses that induce cell fusion include, but are not limited to, HIV, SIV, or respiratory syncytial virus.

For the cell fusion assay, cells are incubated in the presence of a diastereomeric peptide to be assayed. For each peptide, a range of peptide concentrations may be tested. This range should include a control culture wherein no peptide has been added.

Standard conditions for culturing cells, well known to those of ordinary skill in the art, are used. After incubation for an appropriate period, the culture is examined microscopically for the presence of multinucleated giant cells, which are indicative of cell fusion and syncytial formation. Well-known stains, such as crystal violet stain, may be used to facilitate the visualization of syncytial formation.

Alternatively or additionally, cell fusion may be detected by fluorescent dye transfer between labeled donor cells such as, for example, cells expressing HIV-1 gp120-41 and acceptor cells such as, for example, mouse fibroblasts, labeled with a different fluorescent dye. Addition of a diastereomeric peptide of the present invention inhibits dye transfer, which is indicative of inhibition of cell fusion (see Example 1 herein below).

Other assay to evaluate the inhibitory activity of a diastereomeric peptide in membrane protein assembly may use the ToxR system, which is a robust method for detecting homodimerization of transmembrane domains *in* vivo (see Example 3 herein below).

The term "anti-viral" activity refers to the ability of a peptide to inhibit viral infection or transmission of cells via, for example, cell-cell fusion or free virus infection. Assays to test anti-viral activities of a diastereomeric peptide may be based upon measuring an enzymatic activity of a virus as a function of viral infection. If taking HIV as an example, a reverse transcriptase (RT) assay may be utilized to test a peptide ability to inhibit infection of CD-4[+] cells by cell-free HIV. Such an assay may comprise culturing an appropriate concentration (i.e., $TCID_{50}$) of virus and CD-4[+] cells in the presence of the diastereomeric peptide to be tested. Culture conditions well known to those in the art are used. A range of peptide concentrations may be used, in addition to a control culture wherein no peptide has been added. After incubation for an appropriate period of culturing, a cell-free supernatant is prepared, using standard procedures, and tested for the presence of RT activity as a measure of successful infection. The RT activity may be tested using standard techniques (see Goff, S. et al., 1981, J. Virol. 38:239-248; Willey, R. et al., 1988, J. Virol. 62:139-147).

Standard methods, which are well known to those of skill in the art, may be utilized for assaying non-retroviral activity. See, for example, Pringle et al. (Pringle, C. R. et al., 1985, J. Medical Virology 17:377-386) for a discussion of respiratory syncytial virus and parainfluenza virus activity assay techniques.

Methods for assaying the ability of a diastereomeric peptide to modulate intracellular processes involving membrane protein assembly such as, for example, channel formation, nutrient transport, and hormone signaling are well known in the art (see, for example, Falke, J. J., et al., 2000, Curr. Opin. Struct. Biol. 10: 462-469; Pakula, A. A., et al., 1992, Proc. Natl. Acad. Sci. USA 89: 4144-4148; MacKenzie, K. R, et al., 1997, Science 276: 131-133, the content of which is incorporated by reference as if fully set forth herein). An example of the inhibitory activity of nutrient transport in bacteria by a diastereomeric peptide is disclosed herein below (Example 4).

In vivo assays may also be utilized to test, for example, the antiviral activity of the diastereomeric peptides of the invention. To test for anti-HIV activity, for example, the in vivo model described in Barnett et al. may be used (Barnett, S. W. et al., 1994, Science 266:642-646, the content of which is incorporated by reference as if fully set forth herein).

The diastereomeric peptides of the invention may be utilized as anti-fusogenic, anti-viral or anti-chemotactic compounds, or as compounds, which modulate intracellular processes involving functional assembly of membrane proteins such as, for example, channel formation, nutrient transport, and hormone signaling. The diastereomeric peptides may be useful against Gram-positive bacteria such as, for example, Staphylococcus aureus, enterococci, and pneumococci and against Gram-negative bacteria such as, for example, Escherichia coli and Acinetobacter calcoaceticus.

The anti-fusogenic capability of the diastereomeric peptides of the invention may additionally be utilized to inhibit or treat/ameliorate symptoms caused by processes involving membrane fusion events. Such events may include, for example, virus transmission via cell-cell fusion, and sperm-egg fusion. Further, the diastereomeric peptides of the invention may be used to inhibit free viral infection or transmission of uninfected cells wherein such viral infection involves cell-cell fusion events or involves fusion of a viral structure with a host cell membrane.

Retroviral viruses whose transmission may be inhibited by the diastereomeric peptides of the invention include, for example, human retroviruses, particularly HIV-1 and HIV-2, and the human T-lymphocyte viruses (HTLV-I and II). The non-human retroviruses whose transmission may be inhibited by the diastereomeric peptides of the invention include, but are not limited to, bovine leukosis virus, feline sarcoma and leukemia viruses, simian immunodeficiency virus, and sheep progressive pneumonia viruses.

Non-retroviral viruses whose transmission may be inhibited by the diastereomeric peptides of the invention include, but are not limited to, human respiratory syncytial virus, human parainfluenza virus, influenza viruses, measles viruses, Epstein-Barr viruses, and hepatits B viruses. Non-human non-retroviral viruses that may be inhibited by the diastereomeric peptides of the invention include, but are not limited to, simian Mason-Pfizer viruses, canine distemper virus, and Newcastle disease virus.

Non enveloped viruses whose transmission may be inhibited by the diastereomeric peptides of the invention include, but are not limited to, picornaviruses such as polio viruses, hepatitis A virus, enterovirus, echoviruses and coxsackie viruses, papovaviruses such as papilloma virus, parvoviruses, adenoviruses and reoviruses.

The anti-viral activity of the peptides of the invention may show a pronounced type and subtype specificity, i.e., specific diastereomeric peptides may be effective in inhibiting the activity of only specific viruses. This feature of the invention presents many advantages. One such advantage, for example, lies in the field of diagnostics, wherein one can use the antiviral specificity of the diastereomeric peptide of the invention to ascertain the identity of a viral isolate.

Among the intracellular disorders involving functional assembly of membrane proteins, which may be ameliorated by the diastereomeric peptides of the invention are disorders involving, for example, bacterial toxins, overexpression of receptors such as in cancer, and viral infections.

According to another aspect, the present invention provides a pharmaceutical composition comprising a therapeutically effective amount of a membrane binding diastereomeric peptide according to the principles of the present invention and a pharmaceutically acceptable carrier, the diastereomeric peptide capable of inhibiting functional assembly of a membrane protein.

A pharmaceutical composition useful in the practice of the present invention typically contains a diastereomeric peptide of the invention formulated into the pharmaceutical composition as a pharmaceutically acceptable salt form. Pharmaceutically acceptable salts may be prepared from pharmaceutically acceptable non-toxic acids, including inorganic and organic acids. Such acids include acetic, benzenesulfonic, benzoic, camphorsulfonic, citric, ethanesulfonic, fumaric, gluconic, glutamic, hydrobromic, hydrochloric, isethionic, lactic, maleic, malic, mandelic, methanesulfonic, mucic, nitric, pamoic, pantothenic, phosphoric, succinic, sulfuric, tartaric, p-toluenesulfonic acid, and the like.

Pharmaceutically acceptable salts may be prepared from pharmaceutically acceptable non-toxic bases including inorganic or organic bases. Salts derived from inorganic bases include aluminum, ammonium, calcium, copper, ferric, ferrous, lithium, magnesium, manganic, manganous, potassium, sodium, zinc, and the like. Salts derived from pharmaceutically acceptable organic non-toxic bases include salts of primary, secondary, and tertiary amines, substituted amines including naturally occurring substituted amines, cyclic amines, and basic ion exchange resins, such as arginine, betaine, caffeine, choline, N,N'-dibenzylethylenediamine, diethylamine, 2-diethylaminoethanol, 2-dimethylaminoethanol, ethanolamine, ethylenediamine, N-ethyl-morpholine, N-ethylpiperidine, glucamine, glucosamine, histidine, hydrabamine, isopropylamine, lysine, methylglucamine, morpholine, piperazine, piperidine, polyamine resins, procaine, purines, theobromine, triethylamine, trimethylamine, tripropylamine, tromethamine, and the like.

A therapeutically effective amount of a peptide of the invention is an amount that when administered to a patient is capable of exerting an inhibitory activity of functional assembly of a membrane protein and hence of membrane fusion events such as, for example, viral infection, bacterial infection, and intracellular processes involving protein membrane assembly. According to some embodiments, a pharmaceutical composition of the present invention is useful for inhibiting a viral disease in a patient as described further herein. According to such embodiments, a therapeutically effective amount is an amount that when administered to a patient is sufficient to inhibit, preferably to eradicate, a viral disease.

The preparation of pharmaceutical compositions, which contain peptides as active ingredients, is well known in the art. Typically, such compositions are prepared as injectable, either as liquid solutions or suspensions. However, solid forms, which can be suspended or solubilized prior to injection, can also be prepared. The preparation can also be emulsified. The active therapeutic ingredient is mixed with inorganic and/or organic carriers, which are pharmaceutically acceptable and compatible with the active ingredient. Carriers are pharmaceutically acceptable excipients (vehicles) comprising more or less inert substances that are added to a pharmaceutical composition to confer suitable consistency or form to the composition. Suitable carriers are, for example, water, saline, dextrose, glycerol, ethanol, or the like and combinations thereof. In addition, if desired, the composition can contain minor amounts of auxiliary substances such as wetting or emulsifying agents, pH buffering agents, stabilizers, and antioxidants, which enhance the effectiveness of the active ingredient.

The pharmaceutical composition can be delivered by a variety of means including intravenous, intramuscularly, infusion, oral, intranasal, intraperitoneal, subcutaneous, rectal, topical, or into other regions, such as into synovial fluids. However delivery of the composition transdermally is also contemplated, such by diffusion via a transdermal patch.

According to another aspect the present invention provides a method for inhibiting membrane protein assembly in a cell comprising contacting the cell with an effective amount of a membrane binding diastereomeric peptide according to the principles of the present invention, thereby inhibiting membrane protein assembly.

According to a further aspect, the present invention provides a method for inhibiting infection of a cell by a virus comprising contacting the cell with an effective amount of a membrane binding diastereomeric peptide according to the principles of the present invention, thereby inhibiting the infection of the cell.

According to still a further aspect, the present invention provides a method for inhibiting virus replication and transmission in a subject comprising administering to the subject a therapeutically effective amount of a pharmaceutical composition comprising a diastereomeric peptide according to the principles of the present invention dispersed in a pharmaceutically acceptable carrier.

Patients in which the inhibition of viral replication would be clinically useful include patients suffering from diseases transmitted by various viruses including, but not limited to, diseases transmitted by HIV-1 and HIV-2, human respiratory syncytial virus, human parainfluenza virus, measle virus, Epstein-Barr virus, hepatitis B virus, and polio virus. It should be appreciated that Epstein-Barr virus is a human herpes virus, which is the causative agent of, for example, infectious mononucleosis (MN), and is also associated with nasopharyngeal carcinomas (NPC), Burkitt's lymphoma, and other diseases. Additionally, disorders involving bacterial toxins may also be treated/ameliorated by the pharmaceutical composition comprising a diastereomeric peptide of the invention.

The composition is administered in a manner compatible with the dosage formulation, and in a therapeutically effective amount. The quantity to be administered depends on the subject to be treated, and the capacity of the subject's blood hemostatic system to utilize the active ingredient. Precise amounts of active ingredient required to be administered depend on the judgment of the practitioner and are peculiar to each individual.

Methods of treating a disease according to the invention may include administration of the pharmaceutical compositions of the present invention as a single active agent, or in combination with additional methods of treatment. The methods of treatment of the invention may be in parallel to, prior to, or following additional methods of treatment.

Having now generally described the invention, the same will be more readily understood through reference to the following examples, which are provided by way of illustration and are not intended to be limiting of the present invention.

**EXAMPLE 1**

**DP178 Diastereomer**

**Peptides Synthesis**

DP178 diastereomeric analogs were synthesized by using the Boc chemistry described by Merrifield et al. (Merrifield, R. B., et al., 1982, Biochem. 21: 5020-5031). Peptides were cleaved from the resins by HF, precipitated with ether and then purified by reverse-phase HPLC on an analytical C18 Vydac column 4.6 mm X 250 mm (pore size of 300 A). The peptides were eluted by a linear gradient of 25-80% acetonitrile in water containing 0.05% TFA (v/v), at a flow rate of 0.6 ml/min for 80 minutes. Concentrations were measured by tryptophan and tyrosine absorbance (at 280 nm) in 8M Urea.

**Dye transfer fusion assay**

Peptide inhibition of cell-cell fusion was assayed by monitoring the redistribution of two fluorescent probes: a water-soluble probe and a lipophilic probe, between target and effector cells upon their co-incubation with these probes (Munoz-Barroso L, et al., 1998, J. Cell Biol. 140: 315-323). The HIV-1 gp120-41 expressing TF228 cells (Jonak, Z. L., et al., 1993, AIDS Res. Hum. Retrovir. 9:23-32) were labeled with either calcein or a green fluorescent fatty acid as follows: HIV-1 gp120-41 expressing TF228 cells were incubated with 1 $\mu$M calcein for 60 minutes at 37°C, and then washed and resuspended at $10^5$ cells/ml in RPMI medium. Alternatively, the HIV-1 gp120-41 expressing TF228 cells were labeled with the fatty acid 4,4-difluoro-5-methyl-4-bora-3a, 4a-diaza-s-indacene-3-dodecanoic acid (C1-BODIPY-C12; (Molecular Probes, Eugene, OR)) as follows: HIV-1 gp120-41 expressing TF228 cells were seeded at $10^5$ cells/ml in RPMI medium containing 10 mg/ml of the C1-BODIPY-C12 and grown for three days prior to the experiment. The fatty acid was eventually metabolized into phospholipid, primarily phosphatidylcholine. The cells where then washed and resuspended at $10^5$ cells/ml. The target cells, CD4+ CXCR4+ 3T3 mouse fibroblasts, were plated at $10^5$ cells/ml the night before the experiment. The next day, the CD4+ CXCR4+ 3T3 cells were labeled with 20 $\mu$M [5-(and-6) ((4-Chloromethyl)benzoyl)amino)) tetramethylrhodamine (CMTMR) for one hour at 37°C, washed several times and combined with the effector cells (1:3 target-effector cell ratio). Different concentrations of peptides, dissolved in PBS or Tris buffer, were then added. The cells were co-cultured for 2 hours at 37°C in 12 well plates (Costar, Cambridge, MA). Phase and fluorescent images were collected using an Olympus IX70 coupled to a CCD camera (Princeton Instruments, Trenton, NJ) with a 20X objective lens. An 82000 optical filter cube (Chroma Technology Corp., Brattleboro, VT) was used for the excitation of calcein (494/517), BODIPY (500/510), and CMTMR (541/565). Three images per well were collected and then analyzed using metamorph software (Universal Imaging, WestChester, PA) for dye transfer from the donor to the acceptor cell. The scoring of fusion events was conducted as described by Munoz-Barroso et al. (ibid.).

**Results**

**Inhibition of cell-cell fusion induced by DP178 diastereomer**

There are two possible targets for inhibition by DP178: (i) DP178 inhibits the formation of the heterotrimeric coiled-coil gp41 by interacting with the leucine/isoleucine zipper sequence in aqueous solution via its N-terminal region; (ii) fusion pore formation is inhibited by the interaction of C-terminal part of DP178 with another target site within the membrane.

In an attempt to correlate between the structure and the inhibitory activity of DP178, two diastereomeric peptides were synthesized:

(i) A diastereomer of DP178 having two adjacent D-amino acid residues at the N-terminal region of DP178, D-$S^{649}$, $Q^{650}$-DP178 (designated herein D-SQ) having the following amino acid sequence (the D-amino acid residues are bold and underlined):

<div align="center">

YTSLIHSLIEE**SQ**NQQEKNEQELLELDKWASLWNWF     (SEQ ID NO:2)

</div>

Using structurally constrained analogs, McDowell and coworkers demonstrated that the part of C34 having the sequence set forth in SEQ ID NO:26: WMEWDREINNYTSLIHSLIEESQNQQEKNEQELL (corresponding to amino acids 628-661 and overlapping the N-terminal region of DP178) and DP178 inhibit infectivity by adopting a helical conformation (Judice, J. K., et al., 1997, Proc. Natl. Acad. Sci. USA 94: 13426-13430). This occurs when the inhibitor interacts with the coiled-coil forming sequence in aqueous solution. It was therefore expected that D-S649, $Q^{650}$-DP178 would not be able to inhibit HIV-1 induced membrane fusion.

(ii) A diastereomer of DP178 having two adjacent D-amino acid residues at the C-terminal region of DP178, D-$L^{669}$, $W^{670}$-DP178 (herein designated D-LW) having the following amino acid sequence:

<div align="center">

YTSLIHSLIEESQNQQEKNEQELLELDKWAS**LW**NWF     (SEQ ID NO:3)

</div>

It was postulated that if this diastereomer is an inhibitor for HIV-1 infection, it would indicate that the dominant target site of the C-terminal region of DP 178 is within the membrane.

FIG.1 shows the effect of [D-LW]-DP178 on dye transfer fusion assay between HIV-1 gp120-41 expressing TF228 cells and CD4+ CXCR4+ 3T3 mouse fibroblasts. As shown in FIG. 1, [D-LW]-DP178 inhibited the fusion of TF228 cells with 3T3 fibroblasts. Under similar experimental conditions, [D-SQ]-DP178 was completely inactive. Remarkably, [D-LW]-DP178 was more active than the wild-type DP178.

**EXAMPLE 2**

**HIV-1 Fusion Peptide Diastereomer**

We have synthesized a diastereomeric peptide, representing the N-terminal 33 segment of gp41 of HIV-1 (LAV1a) in which four D-amino acid residues IFFA were incorporated. The peptide has the following amino acid sequence:

AVG**I**GAL**F**LG**F**LG**A**AGSTMGARSMTLTVQARQL    (SEQ ID No: 5)

**Results**

**Inhibition of cell-cell fusion induced by the wt-fusion peptide and its diastereomeric analog.**

The diastereomeric and the wild type peptides were tested for inhibitory effect on HIV-1 mediated cell-fusion as described herein above (Example **1**). The diastereomer exhibited significant inhibitory effect already at a concentration of 10 ng/ml (FIG. **2**). An 8-mer N-tenrninal region of the fusion peptide was used as a control for the specificity of HIV-1 inhibition. This control peptide was completely devoid of inhibitory effect, suggesting that the inhibition of the diastereomeric analog of the fusion peptide is specific for HIV-1. It should be indicated that the inhibition exerted by the diastereomeric peptide was identical to that of the wild-type (wt) fusion peptide. Also, a 13-mer N-terminal region of the fusion peptide having the following sequence: AVGIGALFLGFLG set forth in SEQ ID NO: 27 as well as the fusion peptide of Sendai virus having the sequence set forth in SEQ ID NO:28: FFGAVIGTIALGVATSAQITAGIALAEAREAKR, were completely devoid of inhibitory activity, further confirming that the inhibition of the wt-fusion peptide is specific for HIV-1.

**Binding of wt-fusion peptide and its diastereomeric analog to T-cells.**

Nitrobenzofurazane (NBD)-labeled wt-fusion peptide or NBD-labeled diastereomeric fusion peptide (herein designated IFFA) were studied for their binding to activated T-cells as follows: Activated T-cells (10,000/ml) were incubated for 30 minutes at room temperature with NBD labeled wt- or IFFA-fusion peptides (0.5 mM final concentration). The cells were washed twice with PBS (100 ml) in order to remove excess of unbound peptide. The cells were then observed under the fluorescent confocal microscope. NBD excitation was at 488 nm with the laser set at 2% power to prevent bleaching of the fluorophore. Fluorescence data was collected from 525 nm and up. Localization of the peptides to the T-cells was monitored by confocal fluorescence microscopy and is shown in FIG. 3. As shown in FIG. 3, both the wt-fusion peptide and IFFA demonstrated high affinity binding to T-cells. The peptides localized at the plasma membrane and formed patches of high intensity (FIG. **3**), suggesting that both peptides have preferential binding to lipid rafts on the membrane of T-cells. When sliding across the z-axis it becomes obvious that the peptides do not penetrate the cytoplasmic membrane and no peptide was observed within any other membrane compartment in the cells.

**Co assembly of wt-fusion peptide and its diastereomeric analog in the membrane-bound state.**

The self-association of the peptides in their membrane-bound state was monitored by employing resonance energy transfer measurements. Fluorescence resonance energy transfer was measured using NBD-labeled peptides serving as donors and Rho (rhodamine)-labeled peptides serving as energy acceptors. Fluorescence spectra were obtained at room temperature with excitation set at 467 nm using a 10 nm slit width. In a typical experiment, donor peptide (final concentration 0.04 $\mu$M) was added to a dispersion of phosphatidylcholine/cholesterol (PC/Chol; 10:1 w/w) small unilamellar vesicles (SUV; 224 uM) in PBS, followed by the addition of acceptor peptide in several sequential doses. Fluorescence spectra were obtained before and after addition of the acceptor. The efficiency of energy transfer (E) was determined by measuring the decrease in the quantum yield of the donor as a result of the presence of acceptor. E was determined experimentally from the ratio of the fluorescence intensities of the donor in the presence ($I_{da}$) and in the absence ($I_d$) of the acceptor, at the donor's maximum emission wavelength. The percentage of transfer efficiency (E), is given by:

$$E\,(\%) = (1 - I_{da} / I_d) \times 100$$

Correction for the contribution of acceptor emission as a result of direct excitation was made by subtracting the signal produced by the acceptor-labeled analog alone. The contribution of buffer and vesicles was subtracted from all measurements. A dose dependent quenching of the NBD-wt-fusion peptide donor's emission, consistent with energy transfer, was observed when Rho-wt-fusion peptide or Rho-IFFA were added (final concentration of 0.025 $\mu$M to 0.35 $\mu$M) to a mixture of NBD-wt (0.05 $\mu$M) and SUV (400 $\mu$M). The energy transfer was calculated and plotted as a function of the molar ratio of acceptor peptide /lipid (FIG. **4**). The acceptor-peptide was added only after the donor-peptide was already bound to the membrane, thus decreasing association in solution. The lipid/peptide molar ratio in these experiments was kept high in order to create low surface density of donors and acceptors to reduce energy transfer between unassociated peptide monomers. In order to confirm that the observed energy transfer is due to peptide aggregation, the transfer efficiencies observed in the experiments were compared with the energy transfer expected for randomly distributed membrane-bound donors and acceptors (FIG. **4**). The random distribution was calculated as described by Fung and Stryer (Fung, B. K. et al., 1978, Biochem. 17: 5241-5248), assuming that 51 Å is the $R_0$ value for the NBD/Rho donor/acceptor pair. The levels of energy transfer between the different pairs are significantly higher then those expected for random distribution of donors and acceptors. The results clearly demonstrate the ability of the IFFA to associate with the wt-fusion peptide.

## EXAMPLE 3

### Glycophorin-A Diastereomer

Glycophorin A (GPA) has a known homodimerization motif, the core of which is GxxxG. Previous results demonstrated that the GPA transmembrane domain can dimerize in an achiral manner, while preserving wild type like structure and interactions (Gerber, D., et al., 2002, J. Mol. Biol. 322: 491-495). In order to understand the role of the helical secondary structure in the assembly process, a diastereomeric analog (herein designated 2D-GPA) of the GPA transmembrane domain was synthesized. Two L-valines at positions 80 and 84 according to the 1AFO pdb structure, each following a glycine in the GxxxG motif, were replaced by their D-enantiomer. The amino acid sequence of the diastereomeric peptide of GPA is as follows:

ITLIIFG**V**MAG**V**IGT    (SEQ ID NO:11)

Glycines are known to be a weak point for helices and together with the replacement of the large $\beta$-branched valines by their D-enantiomer, they are bound to form a large disturbance in the secondary structure. The ability of the diastereomeric peptide to interact with the wild type GPA transmembrane domain in the membrane of bacteria was tested using the ToxR system (Langosch, D., et al., 1996, J. Mol. Biol. 263: 525-530). In addition, a molecular dynamic simulation was used to try and assess the effect of the structural disturbance on the heterodimer structure formed.

### The diastereomer 2D-GPA hetero-associates with the all-L GPA transmembrane domain

The ToxR GPA plasmid construct is a simple and robust method for detecting homodimerization of transmembrane domains *in vivo* (Langosch, D., et al., 1996, J. Mol. Biol. 263: 525-530). The detection is done through the signal of a $\beta$-galactosidase reporter gene. Heterodimerization can be detected through addition of an exogenous peptide. Interaction of an exogenous peptide with a ToxR GPA receptor within the membrane will form a heterodimer with an inactive ToxR transcription factor. The 2D-GPA peptide of SEQ ID NO:11 was exogenously added to the system in increasing doses (0.5-20 $\mu$M; FIG. **5**). A concentration dependent inhibitory effect was observed. Similar inhibitory effect was also observed with another diastereomeric peptide of SEQ ID NO:12. The inhibition was calculated as follows: I is the inhibitory ability of the peptide, $A_{peptide}$ is the activity of ToxR-GPA in the presence of the peptide, $A_{max}$ is the maximal activity of ToxR-GPA without peptide, and $A_{baseline}$ is the baseline activity of the monomer $A_{16}$ plasmid, a plasmid containing a peptide which consists of 16 amino acids all of which are alanine (Langosch, D., et al., 1996, J. Mol. Biol. 263: 525-530).

$$(1) \qquad I = 1 - \frac{A_{peptide} - A_{baseline}}{A_{max} - A_{baseline}}$$

IC$_{50}$ was calculated by fitting normalized data to the Langmuir equation (equation 3) in the Origin 6.0 commercial program.

$$(2) \qquad AA + B \Leftrightarrow A + AB$$

$$(3) \qquad I = 1 - \frac{k * IC_{50}}{[B] + IC_{50}}$$

Were A represents ToxR-GPA transcription factor, B represents the exogenous peptide, I represents inhibition of beta-galactosidase activity (1-activity) and K is a scaling constant.

The IC$_{50}$ of the interaction between 2D-GPA and the integral GPA transmembrane domain, as extrapolated from the fit, was 0.29 $\mu$M with R$^2$ of 0.95. When fitting previous all L and all D GPA results to this model IC$_{50}$ values of 0.36$\mu$M and 0.48$\mu$M were extrapolated with R$^2$ of 0.92 and 0.95, respectively.

**In vitro recognition between wild type-GPA and the diastereomeric peptide**

Wild type (wt)-GPA and the 2D-GPA analog were synthetically labeled with Rhodamine and NBD, respectively, and fluorescent resonance energy transfer (FRET) experiments were performed with NBD-labeled peptides serving as energy donors and Rhodamine-labeled peptides serving as energy acceptors. Fluorescence spectra were obtained at room temperature with excitation set at 467 nm (8 nm slit) and emission measured at 520 nm (16 nm slits). In a typical experiment, a donor peptide (final concentration 0.1 $\mu$M) was added to a dispersion of large unilamellar vesicles (LUV; 340 $\mu$M) in PBS. This was followed by the addition of an acceptor peptide in increasing doses ranging from 0.01 to 0.05 $\mu$M. At this peptide to lipid ratios, complete membrane binding is expected for all the transmembrane peptides. Fluorescence spectra were obtained before and after addition of the acceptor. The efficiency of energy transfer (E) was determined by measuring the decrease in the quantum yield of the donor as a result of the presence of the acceptor. E was determined experimentally from the ratio of the fluorescence intensities of the donor in the presence (I$_{da}$) and in the absence (I$_d$) of the acceptor, at the wavelengths of the maximal donor's emission. The percentage of transfer efficiency (E), is given by:

$$E\ (\%) = (1 - I_{da} / I_d) \times 100$$

Subtracting the signal produced by the acceptor-labeled analog alone corrected for the contribution of the acceptor emission as a result of direct excitation. The contribution of buffer and vesicles was subtracted from all measurements. The R$_0$ for this pair of donor-acceptor fluorophores is around 51Å. The dose dependent FRET is shown in FIG. **6**. While the fluorescence energy transfer increased when NBD-labeled wt-GPA was used as an acceptor and Rhodamine labeled 2D-GPA was used as a donor, pairs of membrane binding peptides that do not assemble within the membrane showed a much lower energy transfer, as expected.

**ToxR receptor expression levels**

Bacteria FHK$_{12}$ cells were grown in the presence or absence of 2D-GPA, 10$\mu$l samples were added to 2x sample buffer and boiled for 5 minutes. The samples were separated on a 12% SDS-PAGE and thereafter were blotted. The primary antibody used was anti maltose binding protein. The detection was performed with Phototope-HRP Western Blot Detection System (Cell Signaling Technology, MA, USA).

The expression levels of the ToxR GPA construct in FHK$_{12}$ bacteria was compared in the presence or absence of the 2D-GPA of SEQ ID NO:11 as exogenous peptide. As shown in FIG. **7**, no significant differences were detected in the presence of 2D-GPA, thus excluding the possibility that the peptide modifies the expression levels of the ToxR GPA construct. The finding that 2D-GPA did not affect the expression levels of the ToxR GPA construct was further confirmed with another diastereomeric peptide of SEQ ID NO: 12. These results are in agreement with the previously reported results for the all L- and all D-GPA peptides. Therefore, the differences in ToxR signal as observed herein above are a consequence of direct interaction of the receptor with the exogenous peptide within the bacterial membrane.

**Binding of 2D-GPA to the FHK12 bacterial membrane**

The binding of 2D-GPA to the membrane of the FHK12 bacteria is a prerequisite for the interaction between the peptide and the GPA ToxR construct. In order to verify this binding, the bacteria were observed under a confocal microscope in the absence or presence of a NBD labeled 2D-GPA peptide at a 500 nM concentration. Experiments were done as follows: $FHK_{12}$ bacteria were diluted 1:10000 and incubated with NBD labeled 2D-GPA, final concentration of 500 nM, for 30 seconds. Samples were fixed with Glutaraldehyde at a final concentration of 0.001% for 30 seconds, centrifuged and washed 3 times with fresh PBS. Confocal images were obtained using an Olympus IX70 FV500 confocal laser-scanning microscope. Care was taken that any photobleaching did not compromise the interpretation, and laser irradiation and other illumination was prevented between acquisitions. The confocal images were obtained at 12-bit resolution.

The results indicate that the 2D-GPA peptide was localized in the bacterial membrane. This observation was consistent with previously published data for the all L- and all D-GPA peptides. Thus, the present results indicate that the diastereomer 2D-GPA can bind and insert into the bacterial membrane despite of the structural disruption caused by the substitution of the two beta-branched L-valines with their D-enantiomers.

**Molecular dynamic simulation**

Molecular dynamics provides a putative structural rationalization to the observed phenomenon. More specifically, the NMR structure of the all-L GPA homodimer was utilized to produce a heterodimer composed of an all-L GPA peptide and a diastereomeric GPA peptide. Both homodimer and heterodimer were subjected to a molecular dynamics procedure in vacuum to simulate the membrane environment (Gerber, D., et al., 2002, J. Mol. Biol. 322: 491-495). The resulting homodimer and heterodimer structures were compared and analyzed for resemblance (FIG. **8**).

A close look into individual atom contacts of amino acids within the *"interaction motif"* reveals a high similarity. Leu75 and Ile76 form strong inter-chain hydrophobic interactions. Gly79 and Gly83 from chain A have a CA backbone interaction with Va180 and Va184 from chain B. Gly83 from chain A interacts with Gly83 from chain B and the latter interacts also with both Va180 and Va184 from chain B. These interactions confirm that the GxxxG motif, which forms the basis of the knob-into-grove stacking of the two GPA helices, is present in the heterodimer in spite of the two D-Valines. Another strong interaction that drives the dimerization of the helices is the polar-polar interaction between the side chain Thr87 from the two chains.

Overall comparison of the inter-helical contact surface with that of the *"interaction motif"* suggests a major role for the motif in both homodimer and heterodimer structures. The total surface of the inter-helix contact in the resulting structures was $1264 \pm 156 Å^2$ for the homodimer and $1105 \pm 156 Å^2$ for the heterodimer. The surface area of the inter-helix contacts for residues forming the *"interaction motif"* was $782 \pm 72 Å^2$ for the homodimer and $702 \pm 23 Å^2$ for the heterodimer, representing $62 \pm 12$ and $64 \pm 14$ percent out of the total contact surface, respectively. Furthermore, when comparing the contact surface of each residue in the dimer with the opposite helix, it becomes clear that the *"interaction motif"* is playing an important role in the dimerization of both the homodimer and heterodimer (FIG. **8**).

These results indicate that disrupting the secondary structure of the GPA peptide with D-amino acids does not alter significantly the dimerization propensity of the transmembrane GPA. The present findings also imply that "wild type" like dimer structure can be formed within the membrane albeit the large disruption of the secondary structure of one helix. Thus, the membrane is presumably involved in the remarkable preservation of wild type like structure.

**EXAMPLE 4**

**Hetero-assembly between all-L- and a diastereomer of the transmembrane domain-1 of the aspartate Tar receptor**

The *Escherichia coli* aspartate receptor is one of the main chemotaxis receptors found in bacteria and mediates the chemotactic response, mainly to aspartate, glutamate, and maltose. Attractants bind to the periplasmatic region of the receptor and prevent the phosphorylation of a downstream protein, CheY. In the absence of phospho-CheY, the bacteria's motor rotates counterclockwise. This results in a smooth swimming behavior that consequently induces progress toward attaining the high concentration of the attractant.

**Peptide Synthesis and Purification**

The peptides were synthesized by the F-moc solid-phase method on a Rink amide MBHA resin, cleaved from the resin and purified as has been described elsewhere (Sal-Man, N., et al., 2004, Biochem. 43: 2309-2313, the content of which is incorporated by reference as if fully set forth herein). Fluorescent labeling was performed as disclosed herein above (Example 2).

**Results**

Tar-1 all-L peptide corresponding to the transmembrane domain 1 of Tar and a diastereomeric peptides were synthesized. These peptides correspond to the sequence from amino acid 13 to amino acid 28 of Tar-1. The sequences of the peptides and of an inactive mutant FG are as follows:

| | | |
|---|---|---|
| Tar-1 wild type peptide | KKKMVLGVFALLQLISGSLKK | (SEQ ID NO: 20) |
| Tar-1 diastereomer | KKKMVLGVFALL**Q**LISG**S**LKK | (SEQ ID NO: 23) |
| Tar-1 F/G mutant | KKKMVLGVFALLFLIGGSLKK | (SEQ ID NO: 29) |

**Fluorescence Energy Transfer between all-L and all-D Peptides**

To investigate the direct interaction between the two enantiomers of Tar-1 in an isolated *in vitro* system, fluorescence energy transfer (FRET) between fluorescently labeled Tar-1 wild-type peptide and its diastereomeric analog was measured. The assay was performed in a model lipid environment of small unilamellar vesicles (SUV) composed of negatively charged PE/PG lipids (7:3 w/w). This phospholipid composition is typical of the *E. coli* inner membrane. The Tar-1 peptides were labeled with NBD as the donor fluorophore or rhodamine-TAMRA as the acceptor fluorophore, and FRET was measured. The Tar-1 all-L NBD peptide showed about 30% energy transfer in the presence of the Tar-1 diastereomeric rhodamine peptide at an acceptor-to-lipid ratio of 1:7500, indicating an interaction between the two peptides.

**Inhibition of the transmembrane interaction by Tar-1 peptides**

The ToxR transcription activator can be used successfully to assess weak protein-protein interactions within the *E. coli* membrane. A Tar-1 TM domain encoding the DNA cassette was grafted between the ToxR transcription activator and the maltose binding protein in the ToxR-MalE plasmid. The plasmid was then transformed into *E. coli* FHK$_{12}$ cells, which contain β-galactosidase, under the control of a ctx promoter. Dimerization of the TM domains, in this system, results in association and activation of the ToxR transcription activator, which then becomes active and is able to bind the *ctx* promoter. Quantification of the amount of homo-dimerization was done by measuring the activity of the β-galactosidase reporter gene and by normalizing it to the cell content (OD$_{590}$) (miller units)

The ability of the Tar-1 peptides to disrupt the proper dimerization of the wild-type transmembrane (TM) domain was thus investigated by using the ToxR system. This TM assembly system can assess the dimerization of a specific TM domain grafted between the ToxR transcription activator and the maltose binding protein, MalE. Tar-1 TM domain was grafted within the ToxR-MalE chimera protein; it showed marked association activity compared with a control, a GPA construct. In addition, correct integration of the ToxR-TM-MalE chimera proteins into the inner membrane of *E. coli* was examined, and it was found to be inserted in the proper orientation (Sal-Man, N., et al., ibid). The TM-TM interactions were monitored by measuring the activity of β-galactosidase in the presence of different concentrations of exogenous Tar-1 analog peptides. The Tar-1 all-L peptide showed an inhibition profile similar to its diastereomeric counterpart (FIG. **9**), indicating that both peptides interfered with the proper dimerization of the chimera protein TM domain to the same extent.

In order to rule out the possibility that the peptides directly reduced the expression of the chimera protein and not the TM-TM interaction, the expression levels of the chimera proteins were analyzed in the presence of 20μM of each peptide. Western blotting confirmed that the peptides had no effect on the expression levels of the chimera proteins as they remained constant in all samples.

**Effect of the Tar-1 peptides on the chemotaxis response of the Tar receptor**

A plasmid used for overexpression of the wild-type Tar chemoreceptor (pNT201) has been previously described (Borkovich, K. A., et al., 1990, Cell 63: 1339-1348). This receptor-encoding plasmid was maintained in the chemotaxis receptor deleted strain CP362 (Hazelbauer, G., L., et al., 1989, Proc. Natl. Acad. Sci. USA 86: 1448-1452). Swarm assay on TB semi-solid agar plates (0.2% agar containing, except for the negative control, 100 $\mu$g/ml of ampicillin) was carried out at 35°C. Bacteria were incubated with 20$\mu$M Tar-1 all-L, the Diastereomer, and F/G mutant at 35°C for 4hr prior to inoculation on the plates. The growth rate assay was performed by dilution of a CP362-containing pNT201 overnight culture at 1:100. Cell density was measured for 20$\mu$M all-L/ all-D-containing samples as well as in the absence of any peptide at $OD_{650}$ at several time points. Indication of zero growth was obtained from incubation of the bacteria in the presence of chloramphenicol.

The *E. coli* bacteria strain, which overexpresses the Tar protein as the only chemotaxis receptor on the membrane, formed a typical ring on semi-solid agar plates containing Tryptone broth (TB; FIG. **10A**). The same strain, but lacking the plasmid that overexpresses the Tar receptor, did not exhibit any chemotactic behavior, as expected, due to the absence of chemotaxis receptors (FIG. 10B). When the *E. coli* bacteria strain that overexpressed the Tar protein was grown in the presence of 20$\mu$M or Tar-1 all-L or Diastereomer peptides, the expansion speed of the bacteria was significantly reduced (FIG. **10C**). In contrast, 20$\mu$M of the F/G mutant peptide had no significant effect on the chemotactic behavior of the bacteria (FIG. **10D**). In order to rule out the possibility that the inhibitory peptides kill the bacteria or affect their growth rate, the cell density of the bacteria was examined and was shown to be similar both in the presence and in the absence of the peptides (FIG. **10E**).

It will be appreciated by persons skilled in the art that the present invention is not limited by what has been particularly shown and described herein above. Rather the scope of the invention is defined by the claims that follow.

**Claims**

1. A membrane binding diastereomeric peptide comprising from about 7 to 50 amino acid residues corresponding to an amino acid sequence of a fragment of a transmembrane protein, wherein at least two amino acid residues of the diastereomeric peptide are in the D-isomer configuration, said diastereomeric peptide capable of binding the transmembrane protein thereby inhibiting functional assembly of said transmembrane protein, and active fragments, derivatives, analogs or salts thereof.

2. The diastereomeric peptide according to claim 1 comprising from 10 to 40 amino acid residues.

3. The diastereomeric peptide according to claim 1, wherein the membrane protein is selected from the group consisting of viral proteins, bacterial proteins, ion channels, receptors, transporters, and pumps.

4. The diastereomeric peptide according to claim 3, wherein the viral protein is a viral envelope surface glycoprotein.

5. The diastereomeric peptide according to claim 4, wherein the viral envelope surface glycoprotein is selected from the group consisting of envelope surface glycoproteins of HIV, human T-lymphocyte virus, human respiratory syncytial virus, human parainfluenza virus, influenza virus, measles virus, Epstein-Barr virus, bovine leucosis virus, feline sarcoma virus, feline leukemia virus, simian sarcoma virus, simian leukemia virus, simian immunodeficiency virus, canine distemper virus, Newcastle disease virus, simian Mason-Pfizer virus, and sheep progressive pneumonia virus.

6. The diastereomeric peptide according to claim 5, wherein the viral envelope surface glycoprotein is HIV-1$_{LAV1}$ gp41, preferably
   comprising the amino acid sequence of DP 178 set forth in SEQ ID NO:1, preferably
   wherein the diastereomeric peptide,
   is selected from the group consisting of SEQ ID NO:2 and SEQ ID NO:3 or
   further comprising at least one positively charged amino acid residue at the amino terminus, carboxy terminus, or both; or
   comprising the amino acid sequence set forth in SEQ ID NO:4 corresponding to HIV-1$_{LAV1}$ gp41 amino terminal fusion peptide, preferably
   wherein the diastereomeric peptide,
   is selected from the group consisting of SEQ ID NO:5 to SEQ ID NO:7; or further comprising at least one positively charged amino acid residue at the
   amino terminus, carboxy terminus, or both.

7. The diastereomeric peptide according to claim 1, wherein the membrane protein is Glycophorin A, preferably comprising the amino acid sequence set forth in SEQ ID NO: 8, more preferably further comprising at least one positively charged amino acid residue at the amino terminus, carboxy terminus, or both, eve more preferably selected from the group consisting of SEQ ID NO:9 and SEQ ID NO:10,
   Preferably selected from the group consisting of SEQ ID NO:11 to SEQ ID NO:19.

8. The diastereomeric peptide according to claim 3, wherein the bacterial protein is aspartate Tar receptor.

9. The diastereomeric peptide according to claim 8, comprising the amino acid sequence set forth in SEQ ID NO:20 corresponding to the transmembrane-1 domain of the aspartate Tar receptor, preferably further comprising at least one positively charged amino acid at the amino terminus, carboxy terminus, or both;
   preferably selected from the group consisting of SEQ ID NO:22 and SEQ ID NO:23.

10. A pharmaceutical composition comprising as an active ingredient a membrane binding diastereomeric peptide according to any of the preceding claims.

11. A method for inhibiting membrane protein assembly in a cell comprising contacting the cell with an effective amount of a membrane binding diastereomeric peptide according to any one of claims 1 to 9, thereby inhibiting the membrane protein assembly.

12. A method for inhibiting infection by a virus to a cell comprising contacting the cell with an effective amount of a membrane binding diastereomeric peptide according to any one of claims 1 to 13, thereby inhibiting the infection of the cell, wherein the virus is preferably selected from HIV, human T-lymphocyte virus, human respiratory syncytial virus, human parainfluenza virus, influenza virus, measles virus, Epstein-Barr virus, bovine leucosis virus, feline sarcoma virus, feline leukemia virus, simian sarcoma virus, simian leukemia virus, simian immunodeficiency virus, canine distemper virus, Newcastle disease virus, simian Mason-Pfizer virus, and sheep progressive pneumonia virus.

13. A method for inhibiting chemotaxis of a bacterial cell to a nutrient comprising contacting the cell with an effective amount of a membrane binding diastereomeric peptide according to any one of claims 1 to 9, thereby inhibiting the chemotaxis of the bacterial cell to the nutrient.

14. Use of a diastereomeric peptide according to any of the claims 1 to 9 for the preparation of a medicament for inhibiting virus replication or transmission in a subject.

15. The use according to claim 14,
    wherein the subject is a human, preferably wherein the virus is a human virus selected from the group consisting of HIV, human T-lymphocyte virus, human respiratory syncytial virus, human para-influenza virus, influenza virus, measles virus, Epstein-Barr virus, and Hepatitis B virus; or
    wherein the subject is an animal, preferably wherein the virus is selected from the group consisting of bovine leucosis virus, feline sarcoma virus, feline leukemia virus, simian sarcoma virus, simian leukemia virus, simian immunodeficiency virus, canine distemper virus, Newcastle disease virus, simian Mason-Pfizer virus, and sheep progressive pneumonia virus.

FIG. 1

FIG. 2

FIG. 3A          FIG. 3B

FIG. 3C

FIG. 4

FIG. 5

FIG. 6

FIG. 7

FIG. 8

FIG. 9

FIG. 10A

FIG. 10B

FIG. 10C

FIG. 10D

FIG. 10E

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 5464933 A
- US 6133418 A
- US 6093794 A
- US 6228983 B
- US 6017536 B
- US 6013263 B
- US 6020459 B
- US 6020459 A
- US 6518013 B
- US 5840843 A

**Non-patent literature cited in the description**

- **Gerber D. ; Shai Y.** *J. Biol. Chem.,* 2000, vol. 275, 23602-23607
- **Gerber D. ; Shai, Y.** *J. Biol. Chem.,* 2001, vol. 276, 31229-31232
- **Gerber D. ; Shai Y.** *J. Mol. Biol.,* 2002, vol. 322, 491-495
- **Wild C. et al.** *AIDS Res. Hum. Retrov.,* 1993, vol. 9, 1051-1053
- **Lu, M. ; Kim, P. S.** *J. Biomol. Struct. Dynam.,* 1997, vol. 15, 465-471
- **Kliger, Y. et al.** *J. Biol. Chem.,* 2001, vol. 276, 1391-1397
- **Kliger, Y. et al.** *J. Biol. Chem.,* 1997, vol. 272, 13496-13505
- **Pritsker, M. et al.** *Proc. Natl. Acad. Sci. USA,* 1998, vol. 95, 7287-8292
- **Sobolev, V. et al.** *Bioinform.,* 1999, vol. 15, 327-332
- **Benkirane, N. et al.** *J. Biol. Chem.,* 1993, vol. 268, 26279-26285
- **Merrifield.** *J. Am. Chem. Soc.,* 1964, vol. 85, 2149
- **Bodanszky, M.** Principles of Peptide Synthesis. Springer-Verlag, 1984
- **Goff, S. et al.** *J. Virol.,* 1981, vol. 38, 239-248
- **Willey, R. et al.** *J. Virol.,* 1988, vol. 62, 139-147
- **Pringle, C. R. et al.** *J. Medical Virology,* 1985, vol. 17, 377-386
- **Falke, J. J. et al.** *Curr. Opin. Struct. Biol.,* 2000, vol. 10, 462-469
- **Pakula, A. A. et al.** *Proc. Natl. Acad. Sci. USA,* 1992, vol. 89, 4144-4148
- **MacKenzie, K. R et al.** *Science,* 1997, vol. 276, 131-133
- **Barnett, S. W. et al.** *Science,* 1994, vol. 266, 642-646
- **Merrifield, R. B. et al.** *Biochem.,* 1982, vol. 21, 5020-5031
- **Munoz-Barroso L et al.** *J. Cell Biol.,* 1998, vol. 140, 315-323
- **Jonak, Z. L. et al.** *AIDS Res. Hum. Retrovir.,* 1993, vol. 9, 23-32
- **Judice, J. K. et al.** *Proc. Natl. Acad. Sci. USA,* 1997, vol. 94, 13426-13430
- **Fung, B. K. et al.** *Biochem.,* 1978, vol. 17, 5241-5248
- **Gerber, D. et al.** *J. Mol. Biol.,* 2002, vol. 322, 491-495
- **Langosch, D. et al.** *J. Mol. Biol.,* 1996, vol. 263, 525-530
- **Sal-Man, N. et al.** *Biochem.,* 2004, vol. 43, 2309-2313
- **Borkovich, K. A. et al.** *Cell,* 1990, vol. 63, 1339-1348
- **Hazelbauer, G., L. et al.** *Proc. Natl. Acad. Sci. USA,* 1989, vol. 86, 1448-1452